(19) Europäisches Patentamt
European Patent Office
Office européen des brevets

(11) **EP 2 503 938 B1**

(12) **EUROPÄISCHE PATENTSCHRIFT**

(45) Veröffentlichungstag und Bekanntmachung des Hinweises auf die Patenterteilung:
**30.10.2013  Patentblatt 2013/44**

(21) Anmeldenummer: **10787059.4**

(22) Anmeldetag: **23.11.2010**

(51) Int Cl.:
*A61B 5/155* (2006.01)    *A61B 5/151* (2006.01)

(86) Internationale Anmeldenummer:
**PCT/EP2010/067962**

(87) Internationale Veröffentlichungsnummer:
**WO 2011/064178 (03.06.2011 Gazette 2011/22)**

(54) **BANDMAGAZIN MIT RÜCKDREHSPERRE UND INTEGRIERTER BANDFREIGABE**

BELT MAGAZINE WITH BACKING BLOCK AND INTEGRATED BELT RELEASE

MAGAZINE EN BANDES DOTE DE TOURNIQUETS DE RETOUR ET LIBERATION DE BANDE INTEGREE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.11.2009  EP 09176917**

(43) Veröffentlichungstag der Anmeldung:
**03.10.2012  Patentblatt 2012/40**

(73) Patentinhaber:
• **Roche Diagnostics GmbH**
 **68305 Mannheim (DE)**
 Benannte Vertragsstaaten:
 **DE**
• **F.Hoffmann-La Roche AG**
 **4070 Basel (CH)**
 Benannte Vertragsstaaten:
 **AL AT BE BG CH CY CZ DK EE ES FI FR GB GR HR HU IE IS IT LI LT LU LV MC MK MT NL NO PL PT RO RS SE SI SK SM TR**

(72) Erfinder:
• **KONYA, Ahmet**
 **67065 Ludwigshafen (DE)**
• **LIST, Hans**
 **64754 Hesseneck-Kailbach (DE)**
• **KUHR, Hans-Jürgen**
 **68219 Mannheim (DE)**

(74) Vertreter: **Stößel, Matthias**
 **Herzog Fiesser & Partner**
 **Patentanwälte**
 **Dudenstrasse 46**
 **68167 Mannheim (DE)**

(56) Entgegenhaltungen:
**EP-A1- 2 039 293     WO-A1-2007/073912
WO-A1-2009/030359     WO-A1-2009/039926
WO-A2-2005/006985**

**Beschreibung**

Gebiet der Erfindung

Die Erfindung betrifft ein Magazin zum Einsatz in einem analytischen Testgerät sowie ein analytisches Testgerät. Derartige analytische Testgeräte und Magazine werden insbesondere im Bereich der medizinischen Diagnostik eingesetzt, um eine Probe einer Körperflüssigkeit zu generieren und/oder zu sammeln und/oder zu analysieren. Eine Analyse der Probe kann insbesondere eine qualitative oder quantitative Analyse der Probe der Körperflüssigkeit bezüglich eines oder mehrerer Analyte sein. Bei diesen Analyten kann es sich beispielsweise um Metabolite handeln. Ohne Beschränkung weiterer möglicher Einsatzgebiete kann es sich bei diesem Analyten beispielsweise um Blutglukose, Cholesterin, Triglyceride, Koagulate oder ähnliches handeln.

Stand der Technik

Aus dem Bereich der medizinischen Technik, insbesondere der Analytik, sind zahlreiche Testgeräte bekannt, welche analytische Hilfsmittel benötigen. Beispielsweise sind Stechgeräte bekannt, bei denen nacheinander eine Mehrzahl von Lanzetten eingesetzt werden kann, oder analytische Messgeräte, bei welchen nacheinander eine Mehrzahl von Testelementen zum Nachweis mindestens eines Analyten in einer Probe einer Körperflüssigkeit bereitgestellt werden können.

Neben Testgeräten, welche Trommelmagazine oder ähnliche Bereitstellungsvorrichtungen zum Bereitstellen der analytischen Hilfsmittel aufweisen, sind in jüngerer Zeit in zunehmendem Maße Bandgeräte im Einsatz, bei welchen die analytischen Hilfsmittel mittels eines oder mehrerer Trägerbänder bereitgestellt werden. So beschreibt beispielsweise DE 28 03 345 C2 eine Stechvorrichtung zur Blutgewinnung, die ein Bandmagazin umfasst. Bei diesem sind einzelne Lanzetten hintereinander auf einem Band aufgereiht. In ähnlicher Weise beschreibt DE 198 19 407 A1 ein Blutzuckermessgerät mit einem Bandmagazin für Teststreifen. Mit dem Testgerät vom Typ Accu-Chek® Mobile der Roche Diagnostics GmbH ist seit Februar 2009 das erste Blutzuckermessgerät auf dem Markt, welches eine Teststreifen-Bandkassette verwendet.

Der Vorteil des Bandkonzepts, sei es nun für Lanzetten oder für Testelemente, ist in der Regel die relativ große Anzahl von analytischen Hilfsmitteln, beispielsweise Testelementen und/oder Stechelementen, die in einem vergleichsweise kleinen Magazin aufgewickelt vorliegen können. Allerdings stellt sich dabei allgemein das Problem, dass ein Band - im Vergleich zu starren Trägerelementen - in der Praxis vergleichsweise schwierig zu handhaben ist. So sind insbesondere in Bandgeräten einige zusätzliche technische Maßnahmen erforderlich, um eine ordnungsgemäße Führung des Bands zu ermöglichen und beispielsweise ein unkontrolliertes Abwickeln des Bands zu verhindern.

Aus dem Stand der Technik sind so genannte Rückdrehsperren für Bandmagazine bekannt, welche insbesondere beim Herausnehmen des Magazins aus dem Testgerät das Trägerband fixieren können. Hierdurch wird verhindert, dass bereits verbrauchte Testelemente und/oder Stechelemente wieder zum Vorschein kommen, um die Gefahr einer ungewollten Kontamination des Benutzers und/oder anderer Teile des analytischen Testgeräts mit flüssiger Probe zu vermeiden oder um eine Verletzung des Anwenders zu verhindern.

So wird beispielsweise in WO 2006/059232 A1 ein Messgerät mit einem TestsensorenScheibenmagazin beschrieben. Das Scheibenmagazin weist eine Rückdrehsperre auf, welche nur im herausgenommenen Zustand des Magazins aktiv ist, wohingegen diese Sperre im eingesetzten Zustand überbrückt wird.

In WO 2008/022999 A1 wird eine diagnostische Teststreifen-Bandkassette beschrieben. Diese weist eine Drehsicherung mit einer Sperrverzahnung auf, die ein ungewolltes Abwickeln von Testband verhindern soll. Bei einer geräteunabhängigen Handhabung ist diese Sperre wirksam.

In EP 1 690 496 B1 wird ein Blutzuckermesssystem mit einem Teststreifen-Bandmagazin beschrieben. Eine Sperrklinke sorgt dafür, dass sich ein Schlechtwickel, also eine Aufnahmespule für verbrauchte Bandabschnitte des Testbandes, nur in einer Richtung drehen kann. Diese Sperrfunktion ist im eingelegten Zustand des Magazins wirksam.

Auch in WO 2003/071940 A1 wird eine Stechhilfe beschrieben. Diese weist ein Lanzettenmagazin mit einer integrierten Rückdrehsperre auf. Diese Rückdrehsperre soll insbesondere eine Wiederbenutzung der früher benutzten Lanzetten vermeiden.

In WO 03/088835 A2 wird ein System beschrieben, welches auf bandmagazinierten Microsamplern basiert. Auch in diesem System ist eine Rückdrehsperre vorgesehen. Eine Ratschenvorrichtung sorgt dafür, dass ein Bandtransport nur in einer Vorwärtsrichtung erfolgen kann. Auf diese Weise ist das eingelegte Bandmagazin gegen eine versehentliche Rückdrehung gesichert.

Aus EP 2 039 293 A1 ist ein Kombinationsantrieb für ein Probengewinnungssystem zum Gewinnen einer flüssigen Probe bekannt. Das Probengewinnungssystem weist ein Kopplungselement zur Ankoppelung an ein analytisches Hilfsmittel auf sowie eine Antriebseinheit zum Antreiben einer Bewegung des Kopplungselements. Die Antriebseinheit weist weiterhin eine Kopplungsvorrichtung mit mindestens einem drehrichtungssensitiven Element auf, wobei die Kopplungsvorrichtung eingerichtet ist, um in einer ersten Drehrichtung einen Energiewandler an eine erste Systemfunktion und in einer zweiten Drehrichtung an eine zweite Systemfunktion anzukoppeln.

Bei bekannten Testgeräten, welche auf der Verwendung eines Bandmagazins basieren, treten jedoch in der Praxis einige technische Herausforderungen auf. Insbesondere existieren Testgeräte, bei welchen in mindestens einer Applikationsposition mit einem dort befindlichen analytischen Hilfsmittel eine Hubbewegung durchgeführt wird. Diese Hubbewegung kann beispielsweise eine schnell durchgeführte Stechbewegung sein und/oder eine langsam durchgeführte Probennahmebewegung. So kann beispielsweise mittels einer Lanzette eine Perforation einer Hautpartie eines Benutzers erfolgen und/oder es kann mittels eines Testelements in der Applikationsposition flüssige Probe der Körperflüssigkeit abgeholt werden. Eine Problematik bei derartigen Hubbewegungen bandmagazinierter analytischer Hilfsmittel besteht jedoch in einer Bandfreigabe. So ist beispielsweise ein straff gespanntes Trägerband nur bedingt für einen Stech- oder Blutabholvorgang geeignet. Eine Bandfreigabe ermöglicht während der Hubbewegung, bei welcher das Trägerband mitbewegt wird, eine Auslenkung des Trägerbands und vermeidet somit eine Überdehnung, eine bleibende Verformung oder sogar ein Reißen des Trägerbands.

In WO 2009/030359 A1 wird ein Stechsystem mit einem Lanzettenträgerband, das mehrere Lanzetten trägt, beschrieben. Der Stechantrieb bewegt bei einer Stechbewegung eine in die Stechposition gebrachte Lanzette zusammen mit einem diese Lanzette tragenden Abschnitt des Lanzettenträgerbands in Stechrichtung. Mindestens ein in Förderrichtung hinter der Stechposition angeordnetes Teil einer Transporteinrichtung führt, nachdem eine Lanzette in die Stechposition gebracht wurde, vor oder während der Stechbewegung dieser Lanzette eine Bewegung aus. Weiterhin wird vorgeschlagen, dass eine Wickeleinrichtung an der eingelegten Kassette einen Rückwärtsschritt ausführt, um für die jeweilige Stechbewegung Trägerband vom Schlechtwickel abzuwickeln.

Allgemein ist also festzustellen, dass magazinierte Lanzetten, Testelemente oder Microsampler in der Regel eine Rückdrehsperre benötigen, um dem Anwender die erforderliche Hygiene und Sicherheit im Umgang mit dem Medizinprodukt zu geben. Für bandmagazinierte analytische Hilfsmittel sind diese Rückdrehsperren prinzipiell anwendbar. Nachteilig an den bekannten Systemen ist jedoch die Tatsache, dass das Vorhandensein einer Rückdrehsperre in vielen Fällen widersprüchlich ist zu der Forderung nach einer Bandfreigabe, also einer umgekehrten Bandabwicklung während der Hubbewegung, beispielsweise dem Stich oder der Probenabholung. Die Bandfreigabe erfordert in vielen Fällen aber eine Rückdrehung von Trägerband, das bereits am Schlechtwickel aufgewickelt wurde, da ansonsten eine Aktorik der Testsysteme, beispielsweise ein Lanzettengreifer, ungleich belastet würde. Die Konsequenz wäre beispielsweise ein schräger Stich, ein Verrutschen der Lanzette im Greifer, ein Blockieren einer Stechaktuatorik oder ähnliches.

Eine Bandfreigabe ist in vielen Fällen erforderlich, um dem beim Stechvorgang auszulenkenden Trägerband genügend Spielraum zu geben. Hierfür bieten die meisten Lösungen, die aus dem Stand Technik bekannt sind, jedoch kerne Antwort auf die Anwesenheit einer Rückdrehsperre. Eine aktive Rückdrehbewegung des Schlechtwickelantriebs ist in vielen Fällen nicht hinreichend, wenn die Rückdrehsperre des Magazins diese Funktion nicht unterstützt oder andere Alternativen für die Bandfreigabe bietet.

Insbesondere bei Testgeräten und Bandmagazinen, die über eine permanent wirkende integrierte Rückdrehsperre verfügen, lässt sich somit ein Zielkonflikt mit einer Hubbewegung eines Testelements feststellen. Unter einer integrierten Rückdrehsperre ist dabei allgemein eine Rückdrehsperre zu verstehen, welche in das Magazin integriert ist und welche somit eine Rückdrehung verhindern kann, ohne dass hierfür ein Antrieb des Testgeräts erforderlich wäre. Einerseits ist eine derartige integrierte Rückdrehsperre in vielen Fällen wünschenswert, da diese auch bei von dem Testgerät getrenntem Magazin ein unerwünschtes Abwickeln von Trägerband vom Schlechtwickel verhindert. Andererseits besteht jedoch bei Hubbewegungen gerade der Bedarf, gleichmäßig Trägerband vom Gutwickel und vom Schlechtwickel abzuwickeln, um beispielsweise eine spannungsfreie Hubbewegung des Testelements zu ermöglichen.

Die aus dem Stand der Technik bekannten Lösungen, wie beispielsweise die in WO 2006/059232 A1, bei der eine Rückdrehsperre beim Einsetzen des Magazins in das Testgerät überbrückt wird, sind teilweise technisch sehr aufwändig. Derartige Freigaben würden weiterhin in der Regel dazu führen, dass freigegebenes Bandmaterial nach dem Stich lose aufgehängt wäre und gegebenenfalls aus einer Bandführung herausfallen könnte. Eine entsprechend aufwändige Steuerung des Schlechtwickelantriebs könnte diesen Umstand kompensieren.

Aufgabe der Erfindung

Es ist daher eine Aufgabe der vorliegenden Erfindung, ein Magazin und ein analytisches Testgerät bereitzustellen, welche die Nachteile bekannter Magazine und analytischer Testgeräte zumindest weitgehend vermeiden. Insbesondere soll der oben beschriebene Zielkonflikt zwischen einer Rückdrehsperre und einer Bandfreigabe auf technisch einfache Weise gelöst werden.

Offenbarung der Erfindung

Diese Aufgabe wird durch die Erfindung mit den Merkmalen der unabhängigen Patentansprüche gelöst. Vorteilhafte Weiterbildungen der Erfindung, welche einzeln oder in Kombination realisierbar sind, sind in den abhängigen Patentansprüchen dargestellt.

In einem ersten Aspekt der vorliegenden Erfindung wird ein Magazin zum Einsatz in einem analytischen Testgerät vorgeschlagen. Unter einem Magazin ist dabei eine Vorrichtung zu verstehen, welche eine Mehrzahl analytischer Hilfsmittel aufnehmen und bereitstellen kann. Das Magazin kann, wie unten noch näher ausgeführt wird, insbesondere als Bandmagazin ausgestaltet sein, beispielsweise als Bandkassette, und kann allgemein beispielsweise ein Magazingehäuse aufweisen.

Unter einem analytischen Testgerät ist allgemein ein Gerät zu verstehen, welches mindestens eine medizinische Funktion, insbesondere eine analytische und/oder diagnostische Funktion, ausüben kann. Insbesondere kann das analytische Testgerät eine oder mehrere der folgenden Funktionen durchführen: eine Generierung einer Probe einer Körperflüssigkeit, insbesondere durch Perforation einer Hautpartie eines Benutzers; einen Nachweis mindestens eines Analyten in einer Probe einer Körperflüssigkeit, insbesondere einen Nachweis von Glukose und/oder Cholesterin und/oder Triglyceriden und/oder einen Koagulationsnachweis; eine Sammlung einer Probe einer Körperflüssigkeit, insbesondere durch eine Kapillarwirkung.

Das Magazin ist als austauschbares Magazin ausgestaltet. Dies bedeutet, dass das Magazin durch einen Benutzer aus dem analytischen Testgerät entfernt werden kann, um ein frisches, unverbrauchtes Magazin in das analytische Testgerät einzulegen. Beispielsweise kann das analytische Testgerät zu diesem Zweck eine Aufnahme aufweisen, in welche, beispielsweise nach Öffnen eines Gehäuses des Testgeräts das Magazin einlegbar oder einsteckbar ist. Das Magazin ist somit nicht permanent mit dem analytischen Testgerät verbunden. Beispielsweise kann das Magazin selbst über keine oder nur eine unvollständige eigene Aktuatorik verfügen, so dass die oben genannte medizinische Funktion nur in Zusammenwirkung mit dem analytischen Testgerät, welches über eine solche Aktuatorik verfügen kann, ausgeübt werden kann. Weiterhin kann ein Magazingehäuse des Magazins beispielsweise entsprechende Führungen, Nuten, Fixierelemente oder Positionierungshilfen aufweisen, um ein reversibles Einfügen des Magazins in das analytische Testgerät und/oder ein reversibles Anfügen des Magazins an das analytische Testgerät zu ermöglichen.

Das Magazin weist eine Mehrzahl von analytischen Hilfsmitteln auf einem Trägerband auf. Unter einem analytischen Hilfsmittel ist dabei ein Hilfsmittel zu verstehen, welches für mindestens eine medizinische Funktion des analytischen Testgeräts einsetzbar ist. Insbesondere kann es sich dabei um eine Probengenerierungsfunktion und/oder eine Probensammelfunktion und/oder eine Analysefunktion handeln. Dementsprechend können die analytischen Hilfsmittel eines oder mehrere der folgenden analytischen Hilfsmittel aufweisen: eine Lanzette, also ein beliebiges Element zum Durchstechen und/oder Durchschneiden oder allgemein zum Perforieren einer Hautpartie eines Benutzers; ein Element zum Aufnehmen und Transportieren einer Probe einer Körperflüssigkeit des Benutzers, insbesondere eine Kapillare und/oder einen Kapillarspalt; ein Testelement mit mindestens einer Testchemie zum Nachweisen mindestens eines Analyten in der Körperflüssigkeit. Bei der Testchemie kann es sich beispielsweise um ein Material handeln, welches bei Anwesenheit des mindestens einen nachzuweisenden Analyten mindestens eine physikalische und/oder chemisch nachweisbare Eigenschaft ändert. Derartige Testchemien sind aus dem Stand der Technik bekannt. Beispielsweise kann das Testelement ein elektrochemisches und/oder ein optisches Testelement sein. Beispielsweise kann das Testelement mindestens ein Testfeld umfassen. Dabei kann das analytische Magazin derart ausgestaltet sein, dass dieses lediglich eine Art von analytischen Hilfsmitteln bereitstellt. Alternativ können auch mehrere Arten analytischer Hilfsmittel bereitgestellt werden, beispielsweise alternierend. Auch integrierte analytische Hilfsmittel, welche mehrere der genannten Funktionen vereinen, sind möglich. So sind beispielsweise Microsampler bekannt, welche eine Lanzettenfunktion und eine Kapillarfunktion beinhalten und welche ebenfalls als analytische Hilfsmittel im Rahmen der vorliegenden Erfindung einsetzbar sind. Auch integrierte Lanzetten mit Testelementen sind möglich, optional beispielsweise mit einer zusätzlichen Kapillare.

Unter einem Trägerband ist allgemein ein kontinuierlicher Träger zu verstehen, mittels dessen nacheinander analytische Hilfsmittel bereitstellbar sind. Neben einem einfachen Band, beispielsweise einem Papierband, einem Kunststoffband, einem mehrschichtigen Laminatband oder ähnlichen Bändern kommen somit auch andere Arten kontinuierlicher Träger in Betracht, beispielsweise Gliederketten oder ähnliches. Beispielsweise können die analytischen Hilfsmittel auf dem Trägerband angeordnet sein und/oder in das Trägerband integriert sein und/oder auf andere Weise mit dem Trägerband verbunden sein. Die analytischen Hilfsmittel können beispielsweise äquidistant auf dem Trägerband angeordnet sein. So können beispielsweise Lanzetten auf dem Trägerband angeordnet sein und/oder es können Testelemente auf dem Trägerband angeordnet sein, beispielsweise in Form von Testfeldern.

Mittels des Trägerbands sind die analytischen Hilfsmittel in mindestens einer Applikationsposition des Magazins bereitstellbar. Unter einer Applikationsposition ist dabei eine Position zu verstehen, in welcher mindestens eine Funktion des analytischen Testgeräts mit dem analytischen Hilfsmittel, welches sich in dieser Applikationsposition befindet, zusammenwirkt. Beispielsweise kann diese Applikationsposition eine Position sein, in welcher mittels einer Lanzette eine Stechbewegung und/oder eine Probensammelbewegung durchgeführt werden. Alternativ oder zusätzlich kann die Applikationsposition auch eine Position sein, in welcher eine Probennahmebewegung durchgeführt wird. Beispielsweise kann für die Durchführung dieser Funktion eine Aktuatorik des analytischen Testgeräts vorgesehen sein, wie unten noch näher beschrieben wird.

Das analytische Magazin und das analytische Testgerät können beispielsweise derart ausgestaltet sein, dass mittels des Trägerbands die analytischen Hilfsmittel nacheinander in der mindestens einen Applikationsposition bereitgestellt werden. Es können auch mehrere Applikationspositionen vorgesehen sein, beispielsweise eine Applikationsposition für eine Probenaufnahme und eine Applikationsposition für eine Auswertung der Probe. Beispielsweise kann ein Weiterspulen des Trägerbands erfolgen, so dass jeweils ein frisches, bislang noch unbenutztes analytisches Hilfsmittel in der Applikationsposition des Magazins bereitstellbar ist.

Das Magazin weist weiterhin mindestens einen Gutwickel zur Aufnahme von Bereichen des Trägerbands mit unverhrauchten analytischen Hilfsmitteln auf sowie mindestens einen Schlechtwickel zur Aufnahme von Bereichen des Trägerbands mit verbrauchten analytischen Hilfsmitteln. Das analytische Testgerät kann also eingerichtet sein, um mittels eines Transportmechanismus den Schlechtwickel und/oder den Gutwickel derart anzutreiben, dass das Trägerband jeweils derart weitergespult wird, dass ein frisches, unverbrauchtes analytisches Hilfsmittel in der Applikationsposition bereitgestellt wird. Vorzugsweise umfasst das analytische Testgerät somit einen Antrieb, der insbesondere den Schlechtwickel zu einer Drehbewegung antreiben kann, insbesondere zu einer getakteten Drehbewegung. Dieser Weitertransport des Trägerbands wird allgemein im Folgenden auch als "Spulen" bezeichnet. Das Trägerband ist von dem Gutwickel zu dem Schlechtwickel in einer Spulrichtung bewegbar.

Das Magazin weist weiterhin eine Rückdrehsperre des Schlechtwickels auf. Unter dieser Rückdrehsperre, welche ganz oder teilweise in das Magazin integriert sein kann, ist eine Einrichtung zu verstehen, welche eine Rückdrehung des Schlechtwickels verhindert, also eine Bewegung des Trägerbands entgegen der Spulrichtung zumindest so weit, dass ein bereits verbrauchtes, das heißt benutztes, analytisches Hilfsmittel erneut in die Applikationsposition gerät. Die Rückdrehsperre muss also nicht notwendigerweise derart ausgestaltet sein, dass diese eine vollständige Rückdrehung des Schlechtwickels verhindert, sondern kann geringfügige Rückdrehungen erlauben oder sogar, wie unten noch näher aufgeführt wird, begünstigen. Diese Rückdrehung sollte jedoch nicht so weit ermöglicht werden, dass bereits benutzte analytische Hilfsmittel erneut in die Applikationsposition geraten. Alternativ kann die Rückdrehsperre auch derart ausgestaltet sein, dass diese eine vollständige Rückdrehung verhindert.

Wie unten noch näher ausgeführt wird, stellt der Umstand, dass die Rückdrehsperre Bestandteil des Magazins ist einen Vorteil des erfindungsgemäßen Magazins dar, da eine Integration einer Rückdrehsperre in das Magazin dazu führt, dass in dem gebrauchten und entnommenen Magazin, beispielsweise einer gebrauchten Bandkassette, eine Rückabwicklung bereits benutzter analytischer Hilfsmittel auf diese Weise zuverlässig ausgeschlossen werden kann, ebenso wie ein unerwünschtes und unkontrolliertes Abwickeln von Band. Das Magazin weist weiterhin eine Bandfreigabevorrichtung auf, welche eingerichtet ist, um auf der dem Schlechtwickel zuweisenden Seite der Applikationsposition eine Schlechtwickel-Bandreserve des Trägerbands bereitzustellen. Unter einer dem Schlechtwickel zuweisenden Seite der Applikationsposition ist somit ein Teil des Trägerbands zu verstehen, welcher bereits in Spulrichtung die Applikationsposition passiert hat und sich somit auf den Schleditwickel zu bewegt oder bereits auf dem Schlechtwickel aufgewickelt ist.

Die Schlechtwickel-Bandreserve kann insbesondere eine Hubbewegung eines in der Applikationsposition befindlichen analytischen Hilfsmittels ermöglichen, entweder in Alleinstellung oder in Zusammenwirkung mit einer auf der dem Gutwickel zuweisenden Seite der Applikationsposition. Unter einer Bandreserve ist somit allgemein ein Bandabschnitt zu verstehen, der für die Hubbewegung des analytischen Hilfsmittel, einschließlich des mit diesem analytischen Hilfsmittel verbundenen Teils des Trägerbands ermöglicht, ohne dass eine erhebliche Spannung auf das Trägerband ausgeübt wird, welche zu einer Verformung oder sogar zu einem Reißen des Trägerbands führen könnte. Eine Bandreserve ist somit ein Bandabschnitt, das für die Hubbewegung zusätzlich benötigte Trägerband bereitstellen kann. Als Schlechtwickel-Bandreserve wird dabei allgemein die Bandreserve auf der Schlechtwickel-Seite der Applikationsposition bezeichnet, und als Gutwickel-Bandreserve die optionale zusätzliche Bandreserve auf der Gutwickelseite der Applikationsposition.

Dementsprechend kann das Magazin insbesondere derart eingerichtet sein, dass auf der dem Gutwickel zuweisenden Seite der Applikationsposition, was den Gutwickel selbst einschließen kann, zusätzlich eine Gutwickel-Bandreserve des Trägerbands bereitgestellt werden kann. Vorzugsweise ist das Magazin derart eingerichtet, dass die GutwickelBandreserve im Wesentlichen der Schlechtwickel-Bandreserve entspricht. Unter "im Wesentlichen" ist dabei vorzugsweise eine vollständige Übereinstimmung dieser Bandreserven z verstehen, wobei jedoch auch eine Abweichung von nicht mehr als 30%, insbesondere von nicht mehr als 20% und besonders bevorzugt von nicht mehr als 10% im Rahmen dieses Begriffs toleriert werden kann.

So kann beispielsweise das Magazin derart eingerichtet sein, dass die gesamte Bandreserve, die sich aus der Schlechtwickel-Bandreserve und optional der Gutwickel-Bandreserve zusammensetzen kann, vorzugsweise symmetrisch ausgestaltet ist und sich vorzugsweise gleichmäßig aus diesen beiden Bandreserve zusammensetzt. Dadurch können beispielsweise ein Aktor und/oder ein Greifer gleichmäßig belastet werden. Auf diese Weise lassen sich beispielsweise Verkantungen und/oder eine schräge Hubbewegung, beispielsweise ein schräges Stechen, zumindest teilweise vermeiden.

Auf der dem Gutwickel zuweisenden Seite der Applikationsposition kann der Gutwickel selbst die Gutwickel-Bandreserve bereitstellen, da der Gutwickel ohnehin eine Bandfreigabe ermöglichen kann, da bei einer derartigen Bandfreigabe der Gutwickel in seiner gewöhnlichen, beim Weitertakten auftretenden Drehrichtung gedreht wird.

Unter einer Hubbewegung ist dabei allgemein die Bewegung des analytischen Hilfsmittels in der Applikationsposition zu verstehen, welche bei der funktionsgemäßen Verwendung des analytischen Hilfsmittels in dem analytischen Testgerät auftritt. Beispielsweise kann während einer Hubbewegung eine Fixierung des analytischen Hilfsmittels erfolgen, beispielsweise mittels mindestens eines Greifers in der Applikationsposition. Beispielsweise kann es sich bei der Hubbewegung um eine Stechbewegung und/oder eine Probennahmebewegung handeln. Beispielsweise kann mittels eines Greifers des analytischen Testgeräts eine Lanzette zu einer Hubbewegung in Form einer Stechbewegung verwendet werden. Eine derartige Hubbewegung umfasst dann eine schnelle Vorwärtsbewegung, beispielsweise mit einer Spitzengeschwindigkeit von 2 bis 5 m/s, bei welcher eine Perforation der Hautpartie auftritt, gefolgt von einer Rückwärtsbewegung. Während der Rückwärtsbewegung kann auch eine Probenaufnahme erfolgen, beispielsweise mittels eines Kapillarspalts. Alternativ kann die Hubbewegung in Form der Probennahmebewegung eine Vorwärtsbewegung eines Testfeldes hin zu einer Wunde und/oder einer Probe auf einer Hautoberfläche des Benutzers beinhalten, bei welcher kurzfristig das Testfeld bzw. Testelement mit der flüssigen Probe in Kontakt gebracht wird, so dass flüssige Probe auf das Testfeld aufgebracht wird. Diese Probennahmebewegung kann beispielsweise langsam erfolgen, beispielsweise mit einer Spitzengeschwindigkeit von weniger als 1 m/s, beispielsweise mit einer Geschwindigkeit von 0,01 bis 0,5 m/s. Auch hierfür können beispielsweise ein Greifer und/oder auch eine andere Art von Aktor verwendet werden.

Die Hubbewegung eines in der Applikationsposition befindlichen analytischen Hilfsmittels kann allgemein beispielsweise einen maximalen Hub aufweisen, welcher fest vorgegeben sein kann oder welcher auch einstellbar ausgestaltet werden kann. Die Schlechtwickel-Bandreserve kann insbesondere 0,2 bis 0,8 des maximalen Hubs betragen, vorzugsweise 0,5 des maximalen Hubs. Prinzipiell kann die Schlechtwickel-Bandreserve jedoch, je nach Ausgestaltung der Wickel, grundsätzlich alle Werte annehmen zwischen Null und dem maximalen Hub. Dies hängt in der Regel nur von der Art der Bandführung relativ zur Hubrichtung ab und kann beispielsweise in einer Grenzwertbetrachtung veranschaulicht werden. Verläuft das Trägerband beispielsweise senkrecht zur Hubrichtung, dann spielt die Hubbewegung in der Regel kaum eine Rolle. Die benötigte Schlechtwickel-Bandreserve kann nahezu aus der Verschwenkung des Bandes bezogen werden. Verläuft das Band jedoch im Wesentlichen parallel zur Hubbewegung, dann muss in der Regel jede Wickelseite eine komplette Hublänge als Bandreserve zur Verfügung stellen. Der oben angesetzte Wert von 0,5 des maximalen Hubs stellt somit einen in der Praxis häufig anzutreffenden Mittelwert dar. Der maximale Hub kann beispielsweise 0,5 bis 10 mm betragen, insbesondere 1 bis 8 mm und vorzugsweise 3 bis 6 mm.

Die Bandfreigabevorrichtung kann weiterhin derart eingerichtet sein, dass nach der Hubbewegung des analytischen Hilfsmittels die Schlechtwickel-Bandreserve vollständig oder zumindest teilweise wieder aufgenommen wird. Zu diesem Zweck kann die Bandfreigabevorrichtung beispielsweise ein Bandreservoir umfassen, welchem die Schlechtwickel-Bandreserve für die Hubbewegung entnommen werden kann und welchem die Schlechtwickel-Bandreserve nach der Hubbewegung wieder zugeführt werden kann. Dieses Bandreservoir kann beispielsweise, wie unten noch näher ausgeführt wird, in Form mindestens eines beweglich gelagerten Umlenkelements ausgestaltet sein. Auch andere Arten von Bandreservoiren sind jedoch möglich, insbesondere Bandreservoire, welche eine Länge des zwischen der Applikationsposition und dem Schlechtwickel aufgenommenen Teils des Trägerbands variabel gestalten können, um die Schlechtwickel-Bandreserve bereitstellen zu können. Die Bandfreigabevorrichtung kann jedoch, alternativ oder zusätzlich, wie unten noch näher ausgeführt wird, auch ganz oder teilweise in den Schlechtwickel integriert sein. Die Ausgestaltung der Bandfreigabevorrichtung, bei welcher diese weiterhin eingerichtet ist, um nach der Hubbewegung des analytischen Hilfsmittels die Schlechtwickel-Bandreserve wieder aufzunehmen, kann weiterhin auch bewirken, dass ein Verheddern des Bandes durch überschüssiges Band nach der Hubbewegung verhindert wird, was zu einem Verklemmen, einem Verhaken, einem Verrutschen oder einem Verheddern des Trägerbandes führen könnte.

De Bandfreigabevorrichtung ist lediglich auf der Schlechtwickelseite der Applikationsposition vorgesehen, also auf der von der Applikationsposition aus betrachtet dem Schlechtwickel zuweisenden Seite. Auf der dem Gutwickel zuweisenden Seite, zwischen der Applikationsposition und dem Gutwickel ist keine derartige Bandfreigabevorrichtung vorgesehen. Der Gutwickel wirkt hier selbst als Bandfreigabe und stellt die erforderliche Gutwickel-Bandreserve bereit, wenn eine ausreichende Zugspannung auf das Trägerband ausgeübt wird, beispielsweise bei einem Bandtransport und/ oder bei der Hubbewegung, so dass eine zusätzliche Bandfreigabevorrichtung auf der  dem Gutwickel zuweisenden Seite der Applikationsposition vorzugsweise entfallen kann. In anderen Worten wirkt auf der Gutwickelseite der Applikationsposition lediglich der Gutwickel als Bandfreigabevorrichtung, wobei auf der Schlechtwickelseite eine Bandfreigabevorrichtung vorgesehen ist, welche insbesondere zwischen der Applikationsposition und dem Schlechtwickel und/ oder in dem Schlechtwickel selbst angeordnet sein kann. Diese Ausgestaltung bietet gegenüber bekannten Bandführungen den Vorteil, dass auf eine technisch in der Regel ohnehin wirkungslose zusätzliche Bandfreigabevorrichtung auf der Gutwickelseite verzichtet werden kann, was eine technische Vereinfachung, eine Senkung der Herstellungskosten und eine Verringerung des Bauraums bedienen kann. Zudem kann das Band auf diese Weise stets unter einer ausreichenden Spannung gehalten werden, und eine unerwünschte Freigabe von Band durch eine zusätzliche Bandfreigabevorrichtung zwischen der Applikationsposition und dem Gutwickel, welche bis hin zu einem unkontrollierten Abspulen von Band auf der Gutwickelseite führen kann, kann vermieden werden.

Das Magazin kann insbesondere derart ausgestaltet sein, dass ein analytisches Hilfsmittel, welches sich in der Applikationsposition befindet, durch mindestens eine Fixiervorrichtung, insbesondere mindestens einen Greifer, fixierbar ist. Die Fixiervorrichtung kann ganz oder teilweise Bestandteil des Magazins sein, kann jedoch auch ganz oder teilweise in einem analytischen Testgerät enthalten sein, welches das Magazin verwendet und/oder umfasst.

Insbesondere im erfindungsgemässen Fall, in welchem auf der dem Gutwickel zuweisenden Gutwickelseite der Applikationsposition zwischen der Applikationsposition und dem Gutwickel keine Bandfreigabevorrichtung vorgesehen ist und in welchem lediglich auf der Schlechtwickelseite der Applikationsposition mindestens eine Bandfreigabevorrichtung vorgesehen ist, beispielsweise zwischen der Applikationsposition und dem Schlechtwickel und/oder in dem Schlechtwickel, jedoch grundsätzlich auch in anderen Fällen, ist es vorgesehen das jeweils zur Verwendung vorgesehene analytische Hilfsmittel in der Applikationsposition in Transportrichtung fixiert ist oder fixierbar ist. Dies kann beispielsweise durch einen Greifer, beispielsweise den vorstehend genannten Greifer, und/oder eine andere Fixiervorrichtung erfolgen, welche eingerichtet sein können, um ein in der Applikationsposition befindliches analytisches Hilfsmittel kurzfristig derart zu fixieren, dass dieses an einer weiteren Bewegung in Transportrichtung gehindert ist. Diese Fixierung kann beispielsweise während eines für eine Probenentnahme und/oder eine Lanzettenbewegung benötigten Zeitraums erfolgen sowie optional für einen vorgegebenen Zeitraum vor und/oder nach dieser Bewegung. Anschließend kann eine Freigabe erfolgen, beispielsweise indem ein Greifer und/oder eine andere Art von Fixiervorrichtung wieder geöffnet werden, so dass ein Weitertransport ermöglicht wird. Eine derartige Fixierung kann beispielsweise durch eine Wechselwirkung mit einem Antrieb des analytischen Hilfsmittels erfolgen, beispielsweise einem Lanzettenantrieb zur Durchführung einer Lanzettenbewegung und/oder einem Antrieb für ein Testelement zur Durchführung einer Probennahmebewegung, wobei beispielsweise der Antrieb den oben beschriebenen Greifer und/oder die oben beschriebene Fixiervorrichtung umfassen kann.

Bei einer Auslenkbcwegung des Bandes ohne Fixierung des analytischen Hilfsmittels könnte die erforderliche Bandfteigabe in der Regel einseitig vom Gutwickel stammen, da dieser Gutwickel in der Regel die geringere Rückhaltekraft aufweist, beispielsweise aufgrund eines Freilaufs. In der Folge entstünde am analytischen Hilfsmittel eine ungleichmäßige Kraftverteilung, die das Hilfsmittel schief auslenken könnte. Zudem würde möglicherweise die erzwungene Bandfreigabe nicht durch die schlechtwickelseitige Bandfreigabevorrichtung aufgewickelt, da diese in der Regel während des Auslenkvorgangs nicht automatisch mit einlenkt. Es entstünde somit eine zumindest kurzzeitige Bandlose, die erst durch den nachfolgenden Wickelvorgang des Schlechtwickels beseitigt werden könnte. Dies birgt aber die permanente Gefahr, dass das Band nach der Auslenkung aus seiner Führung rutscht und somit unbrauchbar wird. Somit ist in der Regel nur durch eine Fixierung des analytischen Hilfsmittels während der Auslenkung eine symmetrische Bandfreigabe erreichbar.

Die Rückdrehsperre ist, wie oben ausgeführt, derart eingerichtet, dass diese zumindest weitgehend ein Rückdrehen des Schlechtwickels verhindert, bei welchem eine größere Menge an Trägerband mit bereits verbrauchten analytischen Hilfsmitteln eine Bewegung entgegen der Spulrichtung durchführen würde. Ein Rückspulen um geringfügige Strecken entgegen der Spulrichtung, beispielsweise um eine Strecke, welche erheblich kleiner ist als der Abstand zwischen den analytischen Hilfsmitteln auf dem Trägerband, beispielsweise kleiner als 0,8 dieses Abstands, insbesondere kleiner als 0,5 dieses Abstands und vorzugsweise kleiner als 0,3 oder sogar 0,2 dieses Abstands, kann jedoch ermöglicht werden und kann sogar gewünscht sein zum Zweck der Bandfreigabe, wie unten noch näher erläutert wird.

Die Rückdrehsperre ist vorzugsweise eine permanent wirkende Rückdrehsperre, d.h. eine Rückdrehsperre, welche sowohl bei außerhalb des analytischen Testgeräts befindlichem Magazin als auch bei in das analytische Testgerät eingesetztem Magazin wirksam ist. Dies bedeutet insbesondere, dass die Rückdrehsperre vorzugsweise nicht eingerichtet ist, um bei einem Einsetzen des Magazins in dem analytischen Testgerät überbrückt zu werden.

Die Rückdrehsperre kann insbesondere eine oder mehrere der im Folgenden beschriebenen Rückdrehsperren umfassen. So kann die Rückdrehsperre insbesondere mindestens ein drehrichtungssensitives Element umfassen. Insbesondere kann es dabei ein mit dem Schlechtwickel verbundenes drehrichtungssensitives Element umfassen. Unter einem drehrichtungssensitiven Element ist dabei ein Element zu verstehen, welches eine Drehung in einer Richtung ermöglicht und eine Drehung in einer anderen Richtung zumindest weitgehend verhindert. Unter einem zumindest weitgehenden Verhindern ist dabei ein Verhindern zu verstehen, welches vorzugsweise vollständig ist, welches jedoch auch eine geringfügige Rückdrehung umfassen kann, beispielsweise um einen Totwinkel, um den noch eine Rückdrehung möglich ist. Ein derartiger Totwinkel kann sogar, wie unten noch näher erläutert wird, gezielt eingesetzt werden, um die Schlechtwickel-Bandreserve bereitzustellen.

Alternativ oder zusätzlich kann die Rückdrehsperre mindestens einen Freilauf aufweisen, insbesondere mindestens einen mit dem Schlechtwickel verbundenen Freilauf. Unter einem Freilauf ist dabei allgemein eine Vorrichtung zu verstehen, welche in einem Antrieb mit einem Antriebsstrang einen Teil des Antriebsstrangs von einer Drehbewegung entkoppelt, wenn sich Lastverhältnisse ändern. Insbesondere kann der Freilauf eine besondere Ausgestaltung eines drehrichtungssensitiven Elements sein. Ein Freilauf ist in verschiedenen Ausgestaltungen aus dem Stand der Technik bekannt und kann beispielsweise einen Sperrklinkenfreilauf, eine Schlingfeder, einen Klemmkörper- und/oder Klemmrollenfreilauf oder andere Arten von Freiläufen umfassen.

Alternativ oder zusätzlich kann die Rückdrehsperre auch mindestens eine Ratsche umfassen. Unter einer Ratsche ist ebenfalls ein Element mit einer oder mehreren Sperrklinken zu verstehen, welche in entsprechende Gegenstücke eingreifen können, so dass eine Bewegung in einer Richtung ermöglicht wird, eine Bewegung in einer anderen Richtung hingegen verhindert wird. Insbesondere kann die Ratsche mit einem Schlechtwickel verbunden sein. Der oben beschriebene Freilauf kann beispielsweise ein Sonderfall einer derartigen Ratsche sein oder als Ratsche ausgestaltet sein. Allgemein kann das drehrichtungssensitive Element insbesondere mindestens eine Sperrklinke umfassen, insbesondere eine mit dem Schlechtwickel verbundene Sperrklinke und/oder eine mit einem Magazingehäuse verbundene Sperrklinke. Auch eine Konstruktion mehrerer Sperrklinken kann vorgesehen sein. Die Sperrklinken könnten einen linearen Antrieb oder auch einen Rotationsantrieb in einer Richtung ermöglichen und in einer anderen Richtung verhindern.

Wiederum alternativ oder zusätzlich kann die Rückdrehsperre auch mindestens eine auf das Trägerband einwirkende Rückdrehsperre beinhalten. So kann die Rückdrehsperre beispielsweise eine auf das Trägerband einwirkende drehrichtungsabhängige Bremse umfassen. Eine derartige drehrichtungsabhängige Bremse bremst das Trägerband bei einer Bewegung entgegen der Spulrichtung und ermöglicht hingegen eine Bewegung in Spulrichtung. Das Bremsen bei Bewegung entgegen der Spulrichtung kann unmittelbar einsetzen oder erst nach einem konstruktiv bedingten oder vorgegebenen Bremsweg. Die Einwirkung kann dabei unmittelbar auf das Trägerband erfolgen oder mittelbar, beispielsweise indem nicht direkt das Trägerband gebremst wird sondern ein oder mehrere mit diesem Trägerband verbundene analytische Hilfsmittel.

Insbesondere kann eine drehrichtungsabhängige Bremse mittels mindestens einer Walze realisiert werden. Beispielsweise kann ein Walzenspalt zwischen einer Walze und einem Gegenelement, beispielsweise einer zweiten Walze, eingesetzt werden. Beispielsweise können die mindestens eine Walze und/oder die mindestens eine Doppelwalze derart ausgestaltet sein, dass diese ein verformbares Walzenmaterial aufweist. Während eine Bewegung durch den Walzenspalt in Spulrichtung ermöglicht wird, sperrt die Walze bei einer Bewegung entgegen der Spulrichtung, wodurch eine Verformung des Walzenmaterials auftritt, wodurch der Walzenspalt verengt wird und das Trägerband gebremst wird. Insbesondere kann es sich bei dieser mindestens einen Walze somit um eine Gummiwalze handeln.

Wiederum alternativ oder zusätzlich kann auch mindestens ein auf das Trägerband einwirkendes federbelastetes Element eingesetzt werden. Beispielsweise kann eine federbelastete Klappe auf das Trägerband und/oder auf eines oder mehrere der mit dem Trägerband verbundenen analytischen Hilfsmittel einwirken. Das federbelastete Element kann beispielsweise schräg auf dem Trägerband aufliegen, so dass in der Spulrichtung analytische Hilfsmittel das federbelastete Element passieren können, wohingegen entgegen der Spulrichtung die analytischen Hilfsmittel an dem federbelasteten Element verhaken und ein Abbremsen des Trägerbands bewirken können. Das federbelastete Element kann also insbesondere als asymmetrisches federbelastetes Element ausgestaltet sein, welches auf dem Trägerband aufliegt und welches ein Passieren des Trägerbands und/oder der analytischen Hilfsmittel in Spulrichtung ermöglicht, ein Passieren der analytischen Hilfsmittel entgegen der Spulrichtung jedoch verhindert oder zumindest bremst. Das federbelastete Element kann dabei starr ausgebildet sein oder auch verformbar, wobei im letzteren Fall beispielsweise eine Verformung bei einer Bewegung des Trägerbands und/oder der analytischen Hilfsmittel entgegen der Spulrichtung auftreten kann. Die Verformung kann dabei für eine Bremswirkung aufgrund einer erhöhten Krafteinwirkung auf das Trägerband und/oder die analytischen Hilfsmittel sorgen. Unter einer Federbelastung kann dabei eine Belastung des Elements durch eine separate Feder verstanden werden oder auch, alternativ oder zusätzlich, ein Element, welches in sich zumindest teilweise elastische Eigenschaften aufweist, so dass das Element selbst die Federwirkung ausüben kann. Das federbelastete Element kann insbesondere mindestens eine federbelastete Klappe umfassen.

Wiederum alternativ oder zusätzlich kann die Rückdrehsperre auch mindestens eine auf das Trägerband einwirkende Dichtlippe umfassen. Eine Dichtlippe kann auch als Spezialfall des oben beschriebenen federbelasteten Elements ausgestaltet sein, so dass eine Dichtlippe beispielsweise zwei von entgegengesetzten Seiten auf das Trägerband und/oder die analytischen Hilfsmittel einwirkende federbelastete Elemente aufweisen kann. Unter einer Dichtlippe ist dabei ein Element zu verstehen, welches einen Spalt bereitstellen kann, durch welchen das Trägerband mit den analytischen Hilfsmitteln geführt wird. Dabei wirken beidseitig des Spalts verformbare oder in ihrer Position veränderbare Lippen auf das Trägerband und/oder die analytischen Hilfsmittel ein. Die Lippen können auch zumindest abschnittsweise verfonnbare Eigenschaften aufweisen, beispielsweise plastische und/oder elastische Eigenschaften. Bezüglich der Wirkung derartiger verformbarer Eigenschaften kann auf die obige Beschreibung der Wirkung verformbarer federbelasteter Elemente verwiesen werden. Wiederum soll das Einwirken der Dichtlippe derart erfolgen, dass dem Trägerband und/ oder dem analytischen Hilfsmittel ein Passieren der Dichtlippe in Spulrichtung des Trägerbands ermöglicht wird, ein Passieren des Dichtspalts entgegen der Spulrichtung jedoch verhindert oder zumindest gebremst wird. Beispielsweise kann eine Bremswirkung wieder dann eintreten, wenn analytische Hilfsmittel an der Dichtlippe anstoßen. Alternativ oder zusätzlich kann die Bremswirkung derart realisiert werden, dass die Dichtlippe sich verformt, wenn das Trägerband den Spalt der Dichtlippe entgegen der Spulrichtung passiert, so dass der Spalt verengt wird, so dass eine Kraft oder eine erhöhte Kraft auf das Trägerband und/oder die analytischen Hilfsmittel ausgeübt wird.

Das analytische Magazin kann weiterhin optional mindestens eine Bremse aufweisen. Diese Bremse kann eingerichtet sein, um ein Rückspulen des Schlechtwickels und/oder ein Vorwärtsspulen des Gutwickels zu bremsen. Für mögliche Ausgestaltungen dieser optionalen Bremse kann beispielsweise auf US 2006/0240403 A1 verwiesen werden. Auch andere Ausgestaltungen sind jedoch möglich. Besonders bevorzugt wirkt die Bremse nicht unmittelbar auf das Trägerband ein, was gleichwohl möglich ist, sondern wirkt auf den Gutwickel und/oder den Schlechtwickel und/ oder ein mit dem Gutwickel und/oder dem Schlechtwickel mitdrehendes Element ein.

Die Bandfreigabevorrichtung kann auf verschiedene Weisen ausgestaltet sein, um die oben beschriebene Funktion zu realisieren. So kann die Bandfreigabevorrichtung beispielsweise zumindest teilweise in die Rückdrehsperre integriert sein und/oder zumindest teilweise als separates, von der Rückdrehsperre getrennt ausgebildetes Element ausgestaltet sein. Auch eine Mischform dieser Optionen ist denkbar, also eine Form, bei welcher die Bandfreigabevorrichtung teilweise in die Rückdrehsperre integriert ist und teilweise separat und unabhängig von der Rückdrehsperre ausgestaltet ist.

In einer ersten Ausgestaltung ist die Bandfreigabevorrichtung zumindest teilweise, vorzugsweise vollständig, in die Rückdrehsperre integriert. Dies bedeutet, dass die Bandfreigabevorrichtung und die Rückdrehsperre zumindest teilweise bauteilidentisch ausgestaltet sein können.

Dies kann insbesondere derart realisiert werden, dass eine Funktion der Rückdrehsperre von einer Position des Trägerbands abhängig ist. Beispielsweise kann die sperrende oder zumindest bremsende Funktion der Rückdrehsperre bei einer Bewegung des Trägerbands entgegen der Spulrichtung davon abhängig sein, wie die absolute Position des Trägerbands, die absolute Position analytischer Hilfsmittel, die relative Position des Trägerbands relativ zur Rückdrehsperre, die relative Position der analytischen Hilfsmittel und/oder eines bestimmten analytischen Hilfsmittels relativ zur Rückdrehsperre, ein Drehwinkel des Schlechtwickels oder ein Drehwinkel des Gutwickels aktuell eingestellt sind. Eine Position des Trägerbands kann also beispielsweise eine absolute Position, eine relative Position, einen Drehwinkel des Gutwickels oder einen Drehwinkel des Schlechtwickels umfassen.

Die Rückdrehsperre kann dementsprechend eingerichtet sein, um in einer Mehrzahl von Speirpositionen eine Bewegung des Trägerbands entgegen der Spulrichtung zumindest weitgehend zu verhindern oder zumindest zu bremsen, wobei zwischen den Sperrpositionen eine Bewegung des Trägerbands entgegen der Spulrichtung zumindest weitgehend ermöglicht ist, bis eine nächste Sperrposition erreicht ist, wobei bei der Bewegung des Trägerbands entgegen der Spuhrichtung die Schlechtwickel-Bandreserve oder zumindest ein Teil der Schlechtwickel-Bandreserve freigegeben wird.

Diese Mehrzahl von Sperrpositionen, welche auch ausgedehntere Sperrbereiche umfassen können, kann auf verschiedene Weisen realisiert werden. So kann die Rückdrehsperre beispielsweise mindestens ein drehrichtungssensitives Element umfassen, insbesondere ein mit dem Schlechtwickel verbundenes drehrichtungssensitives Element. Dieses soll eine Drehung in einer Richtung ermöglichen und eine Drehung in einer anderen Richtung zumindest weitgehend verhindern. Bezüglich möglicher Ausgestaltungen des drehrichtungssensitiven Elements kann auf die obige Beschreibung verwiesen werden. Unter "zumindest weitgehend verhindern" kann dabei beispielsweise auch noch umfasst sein, das ein Totwinkel vorgesehen ist, um den noch eine Rückdrehung in Sperrrichtung möglich ist. So kann das drehrichtungssensitive Element einen Totwinkel aufweisen und eine Rückdrehung um diesen Totwinkel ermöglichen, wobei das Magazin eingerichtet ist, um bei der Rückdrehung die Schlechtwickel-Bandreserve freizugeben.

Alternativ oder zusätzlich kann die Rückdrehsperre mindestens ein auf das Trägerelement einwirkendes federbelastetes Element aufweisen, insbesondere eine federbelastete Klappe, und/oder mindestens eine Dichtlippe. Dementsprechend kann auf die obige Beschreibung bezüglich der möglichen Ausgestaltungen derartiger Elemente verwiesen werden. Die analytischen Hilfsmittel können bei einer Bewegung des Trägerbands in der Spulrichtung das federbelastete Element passieren, wohingegen bei einer Bewegung des Trägerbands entgegen der Spulrichtung diese analytischen Hilfsmittel und/oder das Trägerband an den genannten Elementen blockieren können oder zumindest gebremst werden können. Die Positionen des Trägerbands, in welchen jeweils ein analytisches Hilfsmittel an dem auf das Trägerband einwirkenden federbelasteten Element und/oder der Dichtlippe anstößt bei einer Bewegung entgegen der Spulrichtung, können somit jeweils Sperrpositionen sein. Befindet sich das analytische Hilfsmitteln hingegen in einer anderen Position, so ist jeweils ein Rückspulen entgegen der Spulrichtung um eine Länge des Trägerbands möglich, solange, bis das nächste analytische Hilfsmittel an dem federbelasteten Element anstößt. Diese Bandlänge kann als Schlechtwickel-Bandreserve genutzt werden.

Ebenfalls wiederum alternativ oder zusätzlich kann die Rückdrehsperre mindestens eine Walze, insbesondere mindestens eine Doppelwalze, aufweisen. Das Trägerband kann durch einen durch die Walze begrenzten Spalt geführt werden. Die Walze kann, wie oben bereits beschrieben, bei einer Bewegung des Trägerbands entgegen der Spulrichtung eine Verformung durchlaufen, wobei durch die Verformung der Spalt verengt wird und eine weitere Bewegung des Trägerbands zumindest erschwert wird. Auch in diesem Fall sind die Rückdrehsperre und die Bandfreigabevorrichtung zumindest teilweise bauteilidentisch.

Die Bandfreigabe kann auch zumindest teilweise unabhängig von der Rückdrehsperre ausgebildet sein. Dies bedeutet, dass die Bandfreigabe zumindest nicht vollständig bauteilidentisch mit der Rückdrehsperre ausgebildet ist und vorzugsweise vollständig als separates Element ausgebildet ist. Auch dies kann auf verschiedene Weisen realisiert werden.

So kann die Bandfreigabe beispielsweise eine beweglich gelagerte Bandumlenkung aufweisen. Eine derartige beweglich gelagerte Bandumlenkung kann beispielsweise ein sogenannter "Tänzer" sein oder einen derartigen Tänzer umfassen, welcher eine mit dem Trägerband in Verbindung stehende Umlenkrolle aufweist, welche beweglich gelagert ist und welche durch mindestens ein Federelement mit einer Kraft gegen das Trägerband beaufschlagt wird. Die beweglich gelagerte Bandumlenkung kann insbesondere zwischen der Applikationsposition und dem Schlechtwickel vorgesehen sein. Die Bandumlenkung kann insbesondere mindestens eine beweglich gelagerte und federbelastete Rolle aufweisen, insbesondere einen "Tänzer" gemäß der obigen Definition.

Die beweglich gelagerte Bandumlenkung kann insbesondere eingerichtet sein, um mindestens drei Positionen einnehmen zu können. Dabei kann insbesondere eine Ruheposition mit einer maximalen Auslenkung der beweglich gelagerten Bandumlenkung vorgesehen sein, also eine maximale Umlenkung des Trägerbands. Weiterhin kann eine Bandtransportposition mit einer mittleren Auslenkung vorgesehen sein. Unter einer mittleren Auslenkung ist dabei nicht notwendigerweise ein geometrisches und/oder arithmetisches Mittel zu verstehen, sondern irgendeine Position zwischen einer maximalen und einer minimalen Auslenkung. Diese Bandtransportposition kann beispielsweise von der beweglich gelagerten Bandumlenkung während eines Weitertaktens des Magazins, insbesondere während eines Spulens des Trägerbands in der Spulrichtung, eingenommen werden. Weiterhin kann eine Hubposition vorgesehen sein, also eine Position, welche von der beweglich gelagerten Bandumlenkung während der Hubbewegung eingenommen werden kann und welche eine minimale Auslenkung aufweist. Neben diesen Positionen können weitere Positionen vorliegen und eingenommen werden.

In einem weiteren Aspekt der vorliegenden Erfindung wird ein analytisches Testgerät vorgeschlagen, welches mindestens ein Magazin gemäß einer oder mehreren der oben beschriebenen Ausgestaltungen aufweist. Das analytische Testgerät ist eingerichtet, um einen Austausch des Magazins zu ermöglichen. Das analytische Testgerät weist weiterhin einen Antrieb zum Antreiben des Trägerbands, insbesondere des Schlechtwickels, auf. Das analytische Testgerät ist weiterhin eingerichtet, um mittels eines in der Applikationsposition befindlichen analytischen Hilfsmittels eine Hubbewegung durchzuführen. Beispielsweise kann das analytische Testgerät zu diesem Zweck mindestens einen Aktor aufweisen, welcher mit dem in der Applikationsposition befindlichen analytischen Hilfsmittel die Hubbewegung durchführt, insbesondere eine Stechbewegung und/oder eine Probennahmebewegung. Beispielsweise kann dieser Aktor mindestens einen Greifer zum Ergreifen des analytischen Hilfsmittels aufweisen und/oder einen Stößel, mittels dessen die Hubbewegung durchgeführt werden kann.

Das Freigeben der Schlechtwickel-Bandreserve durch die Bandfreigabevorrichtung kann insbesondere durch das analytische Testgerät gesteuert werden. So kann das analytische Testgerät beispielsweise mindestens einen Sensor zur Erkennung der Position des Trägerbands aufweisen. Dabei kann es sich, wie oben beschrieben, beispielsweise um eine absolute Position des Trägerbands (beispielsweise eine absolute Position mindestens einer Positionsmarke des Trägerbands) und/oder eine relative Position handeln, beispielsweise eine relative Position, welche ein bestimmtes oder mehrere analytische Hilfsmittel relativ zu einem Messpunkt einnehmen. Beispielsweise kann ein derartiger Sensor derart ausgestaltet sein, dass auf optische, elektrische, mechanische oder akustische Weise ein Eintreffen eines analytischen Hilfsmittels in einer bestimmten Messposition detektiert wird. Auch andere Arten der Detektion sind jedoch möglich. Dementsprechend kann der mindestens eine Sensor entsprechende Sensorelemente zum Erkennen dieses Eintreffens aufweisen. Das analytische Testgerät kann dementsprechend weiterhin eine Steuerung aufweisen, welche eingerichtet ist, um die Schlechtwickel-Bandreserve entsprechend der erkannten Position des Trägerbands einzustellen. Beispielsweise kann diese Steuerung mindestens ein elektronisches Element umfassen, insbesondere mindestens eine Datenverarbeitungsvorrichtung. Auch einfachere Steuerungen sind jedoch möglich, beispielsweise einfache elektronische Trigger, welche eine Freigabe ermöglichen. Unter einem "Einstellen" der SchlechtwickelBandreserve kann beispielsweise verstanden werden, dass ein Weiterspulen des Trägerbands nach Erkennung der Erreichung einer bestimmten Messposition des analytischen Hilfsmittels erfolgt, beispielsweise um einen vorgegebenen Betrag.

Dies kann beispielsweise kombiniert werden mit der obigen Beschreibung der Ausgestaltung des Magazins, bei welchem eine Mehrzahl von Sperrpositionen vorgesehen ist. So kann das Magazin insbesondere, wie oben beschrieben, derart eingerichtet sein, dass eine Funktion der Rückdrehsperre von einer Position des Trägerbands abhängig ist, wobei die Rückdrehsperre eingerichtet ist, um in einer Mehrzahl von Sperrpositionen eine Bewegung des Trägerbands entgegen der Spulrichtung zu verhindern, wohingegen zwischen den Sperrpositionen eine Bewegung des Trägerbands entgegen der Spulrichtung ermöglicht ist, bis eine nächste Sperrposition erreicht ist. Bei der Bewegung des Trägerbands entgegen der Spulrichtung kann dann die Schlechtwickel-Bandreserve freigegeben werden. Ist eine derartige Ausgestaltung des Magazins vorgesehen, so kann die Steuerung eingerichtet sein, um bei Erreichen einer vorgegebenen Sperrposition einen Weitertransport des Trägerbands um die Schlechtwickel-Bandreserve oder einen Betrag, welcher zumindest näherungsweise der Schlechtwickel-Bandreserve entspricht, zu ermöglichen, insbesondere zu veranlassen. Somit kann realisiert werden, dass das Trägerband gerade um die SchlechtwickelBandreserve über die Sperrposition hinaus transportiert wird, so dass ein Rückspulen bis hin zum erneuten Erreichen der Sperrposition möglich wird, wodurch die Schlechtwickel-Bandreserve bereitgestellt wird.

Die erfindungsgemäße Ausgestaltung des Magazins und des analytischen Testgeräts weist im Vergleich zu bekannten Magazinen und analytischen Testgeräten eine Vielzahl von Vorteilen auf. Diese Vorteile ergeben sich insbesondere, wenn die Rückdrehsperre im Magazin bei in das analytische Testgerät eingelegtem Magazin nicht mechanisch überbrückt werden soll, um die Bandfreigabe zu realisieren. Mittels der oben vorgeschlagenen Konzepte lässt sich eine zumindest kleine Schlechtwickel-Bandreserve im Magazin, beispielsweise in einem Magazingehäuse zur Verfügung stellen. Diese Schlechtwickel-Bandreserve kann beispielsweise, wie oben ausgeführt, in etwa der Hälfte des maximalen Hubs der Hubbewegung entsprechen, die beispielsweise das Band bei einem Stechvorgang und/oder einer anderen Art von Hubbewegung ausübt.

Die optionale Gutwickel-Bandreserve kann aus symmetrischen Gründen insbesondere vom Gutwickel entnommen werden, damit beispielsweise ein Lanzettengreifer möglichst gleichmäßig belastet wird. Somit kann vorzugsweise die gesamte Bandfreigabe aufgeteilt werden in die oben skizzierte, mittels der Bandfreigabevorrichtung bereitgestellten Bandreserve auf der Schlechtwickel-Seite von der Applikationsposition betrachtet, und eine Bandreserve auf der Gutwickel-Seite:

$$\text{gesamte Bandfreigabe} = \text{Schlechtwickel-Bandreserve} + \text{Gutwickel-Bandreserve}$$
$$\approx 2 \cdot \text{Schlechtwickel-Bandreserve.}$$

Eine typische Hubbewegung liegt im Bereich zwischen 3 mm und 6 mm, was somit auch ungefähr der benötigten gesamten Bandfreigabe entspricht. Eine bevorzugte Schlechtwickel-Bandreserve, die auf der Schlechtwickel-Seite durch die oben skizzierte Bandfreigabevorrichtung bereitgestellt wird, liegt somit vorzugsweise zwischen 1,5 und 3 mm.

Die Gutwickel-Bandreserve auf der Gutwickel-Seite ist technisch einfach zu realisieren, da der Gutwickel in der Regel einen Freilauf besitzt und bei Übersteigen einer Mindestzugspannung $F_{min}$ in der Regel automatisch Trägerband mit unverbrauchten analytischen  Hilfsmitteln freigibt. Dieses Prinzip wird bereits beim normalen Bandtransport verwendet, wobei der Antrieb des Schlechtwickels in der Regel diese Zugspannung $F_{min}$ aufbringt. Im Falle eines Stechvorgangs muss die Mindestzugspamung durch einen entsprechenden Aktor erreicht werden, um die Gutwickel-Bandreserve auf der Gutwickel-Seite vom Gutwickel abzuwickeln. Beispielsweise kann dieser Aktor einen Greifermechanismus und/oder einen Stechantrieb umfassen. Auf der Seite des Schlechtwickels stellt sich die Situation hingegen, wie oben ausgeführt, bei Magazinen nach dem Stand der Technik ungünstiger dar. Der Antrieb des Schlechtwickels ist im Normalfall in der Regel inaktiv und blockiert damit eine Rückdrehung des Schlechtwickels aufgrund der Rückdrehsperre. Mittels der oben beschriebenen erfindungsgemäßen Lösung, mittels derer sich eine trotz integrierter Rückdrehsperre eine Schlechtwickel-Bandreserve bereitstellen lässt, lässt sich dieses Problem und damit der eingangs dargestellte Zielkonflikt jedoch leicht und ohne größeren technischen Aufwand lösen.

Es lässt sich somit insbesondere eine permanente integrierte Rückdrehsperre in einem Magazin, insbesondere einem Bandmagazin, realisieren. Diese Rückdrehsperre kann einen Anwender an einem versehentlichen Abwickeln von Spannmaterial vom Schlechtwickel hindern, zum Beispiel nach der Entnahme des Magazins aus dem analytischen Testgerät. Die Rückdrehsperre kann somit permanent aktiv sein.

Kurze Beschreibung der Figuren

Weitere Merkmale und Einzelheiten der Erfindung ergeben sich aus der nachfolgenden Beschreibung bevorzugter Ausführungsbeispiele. Die Ausführmigsbeispiele sind in den Figuren schematisch dargestellt. Gleiche Bezugszeichen bezeichnen dabei gleiche bzw. funktionsgleiche oder hinsichtlich ihrer Funktionen einander entsprechende Elemente. Die Erfindung ist nicht auf die Ausführungsbeispiele beschränkt.

Im Einzelnen zeigen:

Figur 1              ein Ausführungsbeispiel einer in eine Rückdrehsperre integrierten Bandfreigabevorrichtung in Form einer drehwinkelabhängigen Sperre;

Figur 2              ein Ausführungsbeispiel einer in eine Rückdrehsperre integrierten Bandfreigabevorrichtung in Form einer federbelasteten Klappe;

Figur 3              ein Ausführungsbeispiel einer in eine Rückdrehsperre integrierten Bandfreigabevorrichtung mit einer Dichtlippe;

Figur 4             ein Ausführungsbeispiel einer in eine Rückdrehsperre integrierten Bandfreigabevorrichtung mit zwei Gummiwalzen;

Figur 5             ein Ausführungsbeispiel einer optionalen Bremse; und

Figuren 6A bis 6C   ein Ausführungsbeispiel einer separat von einer Rückdrehsperre aus-gebildeten Bandfreigabevorrichtung in Form eines Tänzers.

Ausführungsbeispiele

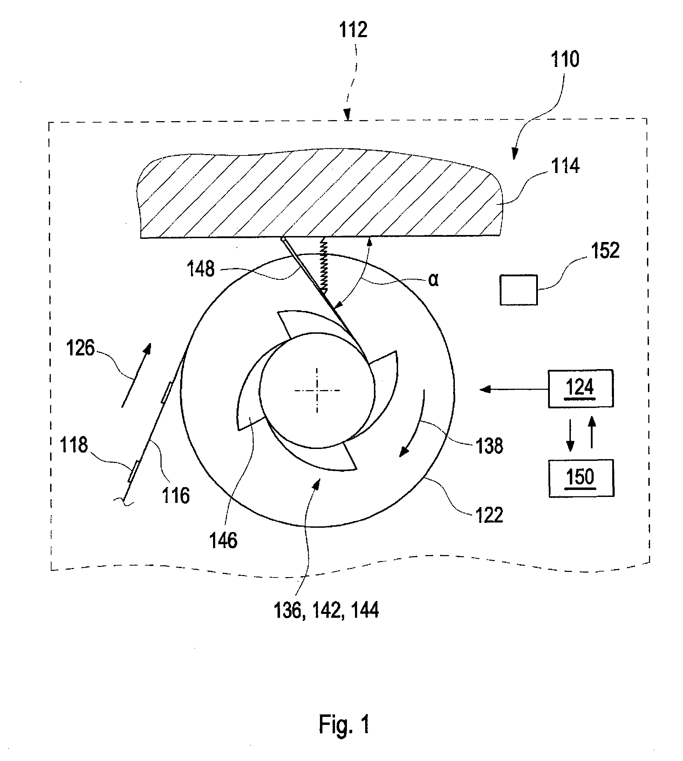

In den Figuren 1 bis 6C sind verschiedene Ausführungsbeispiele eines Ausschnitts eines Magazins 110 zum Einsatz in einem analytischen Testgerät (in den Figuren lediglich angedeutet, Bezugsziffer 112) dargestellt. Das Magazin 110 ist in diesem Ausführungsbeispiel als Bandmagazin ausgestaltet und kann beispielsweise ein in den Figuren lediglich teilweise angedeutetes Magazingehäuse 114 aufweisen. Das Magazin 110 ist als auswechselbares Magazin ausgestaltet und kann dementsprechend ausgetauscht werden.

Das Magazin 110 weist ein Trägerband 116 mit einer Mehrzahl analytischer Hilfsmittel 118 auf. Beispielsweise kann es sich bei diesen analytischen Hilfsmitteln 118 um Testelemente, beispielsweise Testelemente mit mindestens einem Testfeld, und/oder um Lanzetten oder Microsampler handeln. Auch eine Ausgestaltung mit unterschiedlichen Arten analytischer Hilfsmittel 118, beispielsweise Lanzetten und Testelementen, ist denkbar.

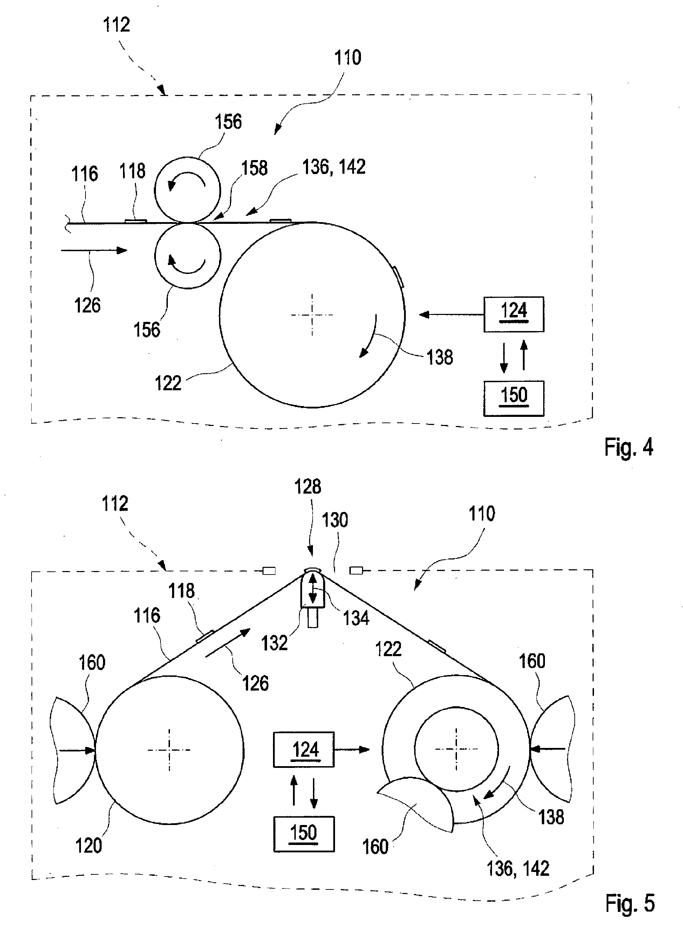

Abschnitte des Trägerbands 116 mit bislang noch nicht benutzten analytischen Hilfsmitteln 118 sind auf einem Gutwickel 120 (siehe beispielsweise die Figuren 5 bis 6C, in den übrigen Figuren nicht dargestellt) aufgewickelt, und Abschnitte mit bereits verwendeten analytischen Hilfsmitteln 118 werden auf einem Schlechtwickel 122 aufgewickelt. Das analytische Testgerät 112 weist beispielsweise einen in den Figuren lediglich angedeuteten Antrieb 124 auf, welcher auf den Schlechtwickel 122 einwirkt, so dass das Trägerband 116 in einer Spulrichtung 126 durch das Magazin 110 gespult werden kann.

Das Spulen erfolgt dabei derart getaktet, dass nacheinander analytische Hilfsmittel 118 in einer Applikationsposition 128 (angedeutet in den Figuren 5 bis 6C) bereitgestellt werden  können. In dieser Applikationsposition 128 können ein Gehäuse des analytischen Testgeräts 112 und/oder das Magazingehäuse 114 einen Durchbruch 130 aufweisen, durch wel-chen hindurch das in der Applikationsposition 128 befindliche analytische Hilfsmittel 118 mit einer Probe außerhalb des analytischen Testgeräts 112 und/oder mit einer Haut eines Benutzers wechselwirken kann. Handelt es sich bei dem analytischen Hilfsmittel 118 um eine Lanzette, so kann es sich bei dieser Wechselwirkung beispielsweise um eine Lanzettenbewegung in Form einer Vorwärtsbewegung mit einem Einstich in die Haut des Benutzers, gefolgt optional von einer Rückbewegung, handeln. Bei der Rückbewegung kann, wenn die Lanzette mit einer Kapillare ausgestattet ist, optional auch eine Aufnahme von Probe durch die Kapillarwirkung erfolgen. Handelt es sich bei dem analytischen Hilfsmittel 118 um ein Testelement, beispielsweise mit mindestens einem Testfeld, so kann es sich bei der genannten Wechselwirkung um eine Vorwärtsbewegung handeln, bei welcher ein Probenaufnahmebereich des Testelements und/oder das Testfeld selbst mit einer, beispielsweise auf einer Hautoberfläche des Benutzers befindlichen Probe der Körperflüssigkeit in Kontakt gebracht wird, um die Probe auf das Testelement aufzubringen. Das Testelement kann ebenfalls über mindestens eine Kapillare verfügen, beispielsweise um die Probe von einer Applikationsstelle oder Probenaufgabestelle zu einem Testfeld und/oder einer anders gearteten Testchemie zu führen.

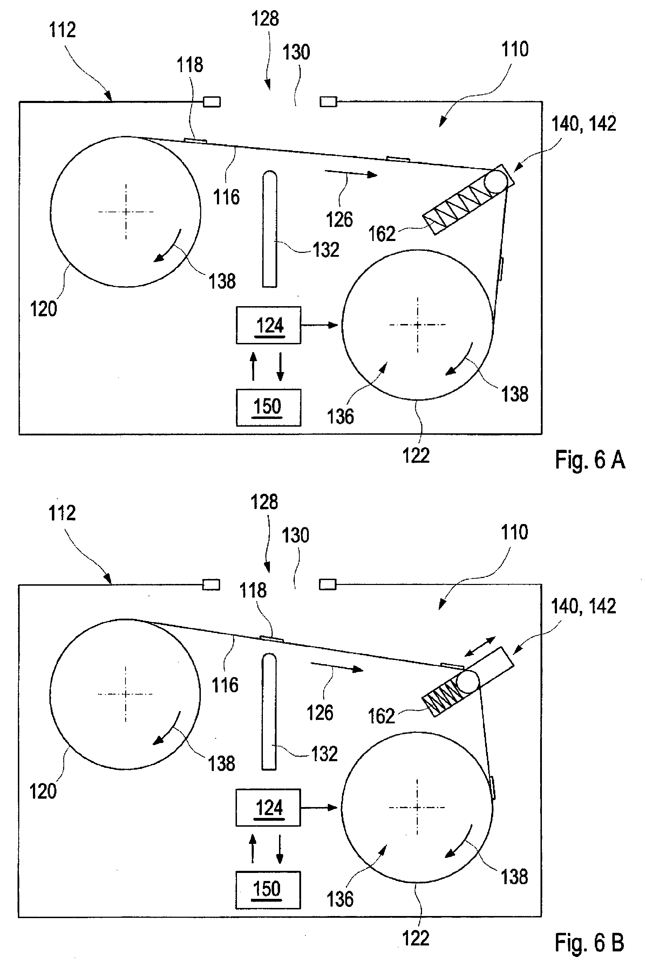

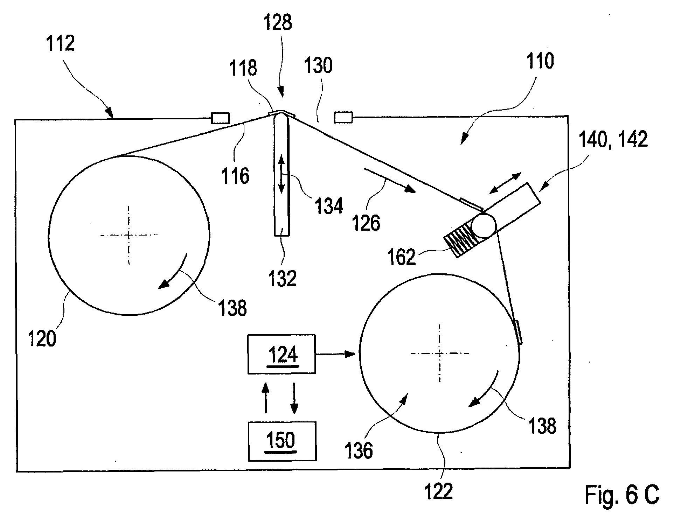

Um diese Wechselwirkung zu gewährleisten, weist das analytische Testgerät 112 in den dargestellten Ausführungsbeispielen vorzugsweise mindestens einen Aktor 132 auf. Dieser Aktor 132, welcher lediglich in den Figuren 5 und 6A bis 6C angedeutet ist, ist eingerichtet, um eine Hubbewegung durchzuführen, welche in den Figuren symbolisch mit der Bezugsziffer 134 bezeichnet ist (siehe Figuren 5 und 6C). Eine derartige Hubbewegung 134 ist somit eine Bewegung hin zu einer Hautoberfläche des Benutzers. Die Hubbewegung 134 ist wiederum auf die Art des analytischen Hilfsmittels 118 anpassbar. Beispielsweise kann es sich bei dieser Hubbewegung um eine schnelle Hubbewegung mit einer Geschwindigkeit von einigen Metern pro Sekunde handeln, sofern die analytischen Hilfsmittel 118 eine Lanzette umfassen. In diesem Fall ist die Hubbewegung als Stechbewegung ausgestaltet, optional begleitet oder gefolgt von einer Probenaufnahmebewegung durch eine Kapillare bei einer Rückwärtsbewegung der Hubbewegung. Umfasst das analytische Hilfsmittel 118 ein Testelement, so ist die Hubbewegung in der Regel eine Probenaufnahmebewegung, welche typischerweise langsam erfolgt, beispielsweise mit Geschwindigkeiten unterhalb von 1 Meter pro Sekunde. Entsprechend kann der Aktor 132, wobei auch mehrere derartige Aktoren 132 vorgesehen sein können, ausgestaltet sein. So kann der Aktor 132 beispielsweise als Stechstößel ausgestaltet sein, um die Lanzettenbewegung durchzuführen. Alternativ kann der Aktor 132 auch als langsamer Probenaufnahmestößel  ausgestaltet sein, um beispielsweise ein Testelement hin zu einer Hautoberfläche des Benutzers zu führen und zurück.

Der Schlechtwickel 122 ist in den dargestellten Ausführungsbeispielen mit einer lediglich angedeuteten Rückdrehsperre 136 ausgestattet. Diese Rückdrehsperre 136 ist somit in das Magazin 110 integriert und wirkt vorzugsweise permanent, d.h. diese Rückdrehsperre 136 wird beim Einsetzen des Magazins 110 in das analytische Testgerät 112 nicht überbrückt oder geschwächt. Derartige Rückdrehsperren sind aus dem Stand der Technik grundsätzlich bekannt. Beispielsweise können zu diesem Zweck drehrichtungssensitive Elemente eingesetzt werden, wie beispielsweise Sperrklinkenräder, Freiläufe oder ähnliches. Diese ermöglichen eine Drehung des Schlechtwickels 122 in einer in Figur 1 angedeuteten Aufwickelrichtung 138, in welcher sich das Trägerband 116 in Spulrichtung 126 bewegt. Eine Drehung des Schlechtwickels 122 in entgegengesetzter Richtung wird hingegen zumindest weitgehend verhindert. Im Gegensatz zu bekannten Probengewinnungssystemen ist im Rahmen der vorliegenden Erfindung jedoch vorgesehen, dass die mindestens eine Rückdrehsperre 136 in das Magazin 110 integriert ist. Auf diese Weise kann das Magazin, 110 einem Gerät, beispielsweise einem Probengewinnungssystem, auch nach zumindest teilweiser Benutzung entnommen werden, wobei zuverlässig verhindert werden kann, dass eine Rückabwicklung und dementsprechend eine Wiederverwendung bereits benutzter analytischer Hilfsmittel 118 oder ein unerwünschtes Abspulen von Trägerband 116 erfolgt. Bei derartigen Magazinen 110 mit einer Rückdrehsperre 136 tritt die oben beschriebene Problematik auf, dass bei der Hubbewegung 136 eine Bandfreigabe erfolgen muss. Diese Bandfreigabe berücksichtigt, dass beim maximalen Hub der Hubbewegung 134 eine zusätzliche Menge an Trägerband 116 benötigt wird, welche auch als Bandfreigabe bezeichnet wird. Diese sollte aus symmetrischen Gründen gleichermaßen vom Gutwickel 120 und vom Schlechtwickel 122 bzw. aus einem Abschnitt des Trägerbands 116 zwischen der Applikationsposition 128 und dem Gutwickel 120 bzw. dem Schlechtwickel 122 entnommen werden. Zwischen diesen Möglichkeiten wird im Folgenden nicht unterschieden, und die Gutwickel-Bandreserve, welche der dem Gutwickel 120 zuweisenden Seite der Applikationsposition 128 entstammt, wird als Bandreserve G bezeichnet und die Schlechtwickel-Bandreserve, welche der dem Schlechtwickel 122 zuweisenden Seite der Applikationsposition 128 entnommen wird, als Bandreserve S. Vorzugsweise sollte dann gelten:

$$\text{gesamte Bandfreigabe} = \text{Bandreserve S} + \text{Bandreserve G} \approx 2 \cdot \text{Bandreserve S.}$$

Wie oben bereits dargestellt, liegt eine typische Hubbewegung 134 bei einem maximalen Hub im Bereich zwischen 3 und 6 mm. Dementsprechend sollten also ca. 3 bis 6 mm an Trägerband 116 als Bandfreigabe vorgesehen sein, so dass sich die Schlechtwickel-Bandreserve des Schlechtwickels auf typischerweise 1,5 bis 3 mm belaufen sollte. Während die Gutwickel-Bandreserve des Gutwickels relativ einfach zu realisieren ist, da der Gutwickel 120 bei der Freigabe dieser Bandreserve G ohnehin in seiner Freigaberichtung gespult wird, stellt sich auf der Seite des Schlechtwickels die Situation ungünstiger dar. Der Antrieb 124 des Schlechtwickels 122 ist im Normalfall während der Hubbewegung 134 inaktiv und blockiert damit in vielen Fällen eine Rückdrehung des Schlechtwickels 122. Möglich wäre zwar eine aktive Rückdrehung des Antriebs 124 zum Zwecke dieser Bandfreigabe, was jedoch technisch vergleichsweise aufwendig ist und was daher im Rahmen der vorliegenden Erfindung vorzugsweise nicht realisiert wird. Gleichwohl ist eine derartige aktive Rückdrehung des Antriebs 124 auch im Rahmen der vorliegenden Erfindung realisierbar. Weiterhin wäre, alternativ oder zusätzlich, auch eine Abkopplung des Antriebs 124 vom Schlechtwickel 122 möglich, beispielsweise über einen zweigeteilten, federgelagerten Schlechtwickel, welcher zumindest einen kleinen Rückdrehschritt entgegen der Aufwickelrichtung 138 unter dem Krafteinfluss des Antriebs des Aktors 132 zulässt. Als dritte mögliche Realisierung einer Bandfreigabe, welche alternativ oder zusätzlich eingesetzt werden kann, kann, wie oben noch näher ausgeführt wird, eine beweglich gelagerte Bandumlenkung umfassen, welche unten anhand der Figuren 6A bis 6C erläutert wird und welche auch als "Tänzer" bezeichnet wird. Derartige Tänzer sind beispielsweise aus einem Tonbandgerät bekannt und können unterschiedliche Zugkräfte am Trägerband 116 ausgleichen.

Diese Konzepte bedingen jedoch eine Bandkassette, bei der der Schlechtwickel 122 die entsprechende Bewegung zur Freigabe der Schlechtwickel-Bandreserve des Schlechtwickels 122 trotz wirksamer Rückdrehsperre 136 vorsieht.

Hierzu werden verschiedene Konzepte vorgeschlagen, welche im Folgenden anhand der Figuren 1 bis 6C erläutert werden sollen. In allen Fällen weist das Magazin 110 eine Bandfreigabevorrichtung 142 auf. Diese Bandfreigabevorrichtung 142 ist eingerichtet, um während der Hubbewegung 134 die Schlechtwickel-Bandreserve des Schlechtwickels 122 Bandreserve S) bereitzustellen. Die in den Figuren 1 bis 6C dargestellten Konzepte lassen sich dabei grundsätzlich in zwei verschiedene Gruppen unterteilen, welche jedoch auch miteinander kombiniert werden können. So sind in den Figuren 1 bis 4 Konzepte gezeigt, bei welchen die Bandfreigabevorrichtung 142 in die Rückdrehsperre 136 integriert ist oder zumindest teilweise bauteilidentisch mit dieser Rückdrehsperre 136 ist. In den Figuren 6A bis 6C ist hingegen ein Konzept gezeigt, bei welchem eine separate Bandfreigabevorrichtung 142 vorgesehen ist, welche unabhängig von der Rückdrehsperre 136 ausgebildet ist.

Vorzugsweise ist eine Bandfreigabevorrichtung 142 ausschließlich auf der SchlechtwickelSeite, betrachtet von der Applikationsposition 128, vorgesehen, wohingegen auf der Gutwickel-Seite, also im Gutwickel 120 und/oder auf einem Bandabschnitt des Trägerbands 116 zwischen Gutwickel 120 und Applikationsposition 128, vorzugsweise keine derartige Bandfreigabevorrichtung 142 vorgesehen ist, insbesondere keine beweglich gelagerte Bandumlenkung 140.

In Figur 1 ist als erstes Ausführungsbeispiel der Bandfreigabevorrichtung 142 eine drehwinkelabhängige Sperre 144 vorgesehen. Diese drehwinkelabhängige Sperre ist eine Rückdrehsperre, 136, die nur bei bestimmten Drehwinkeln wirksam ist und dazwischen einen Schlupf besitzt. Dieser Schlupf muss ausreichend dimensioniert sein, um die Bandreserve S bereitstellen zu können. Dies ist in Figur 1 angedeutet, wobei in diesem Fall auf dem Schlechtwickel 122 Ratschenzähne 146 vorgesehen sind. In diesem Fall sind diese Ratschenzähne 146 gerade um eine Vierteldrehung beabstandet, so dass der Schlupf in dem dargestellten Ausführungsbeispiel maximal 90 Grad beträgt. Auch andere Beabstandungen sind jedoch möglich. Der Schlupf sollte derart dimensioniert sein, dass die Bandreserve S kleiner ist als der Abstand zwischen benachbarten analytischen Hilfsmitteln 118. Diese bevorzugte Ausgestaltung gilt auch für andere Ausführungsbeispiele der vorliegenden Erfindung, so dass allgemein die Bandreserve S vorzugsweise kleiner ist als der Abstand zwischen benachbarten analytischen Hilfsmitteln 118, so dass verhindert wird, dass durch die Bandfreigabevorrichtung 142 ein bereits benutztes analytisches Hilfsmittel 118 wieder in die Applikationsposition 128 zurückgespult werden kann.

Die Ratschenzähne 146 können beispielsweise mit einer oder mehreren Sperrklinken 148 zusammenwirken. Diese können auch federgelagert sein, wie in Figur 1 angedeutet. Die Sperrklinke 148 kann beispielsweise einem Magazingehäuse 114 zugeordnet sein, und die Ratschenzähne 146 dem Schlechtwickel 122. Auch eine umgekehrte Ausgestaltung ist möglich. Wird das Trägerband 116 bis unmittelbar vor dem nächsten wirksamen Ratschenzahn 146 aufgewickelt, so kann der Schlupf bis zum Blockieren des vorhergehenden Ratschenzahns 146 bei der Rückdrehbewegung des Schiechtwickels 122 entgegen der Aufwickelrichtung 138 für die Bandfreigabe verwendet werden. Zu diesem Zweck kann beispielsweise während dieser Zeit eine Rückdrehung des Antriebs 124 erfolgen und/oder eine Abkopplung des Antriebs 124 vom Schlechtwickel 122.

Allgemein kann in diesem oder auch in anderen Ausführungsbeispielen das analytische Testgerät 112 beispielsweise eine in Figur 1 lediglich angedeutete Steuerung 150 umfassen. Diese Steuerung 150 kann beispielsweise eine Steuerlogik umfassen. Die Steuerung 150 kann beispielsweise mit der Ratschenfunktion der Bandfreigabevorrichtung 142 gekoppelt sein. Beispielsweise kann mindestens ein Sensor 152 vorgesehen sein, welcher in Figur 1 angedeutet ist und welcher auch in den übrigen Ausführungsbeispielen vorgesehen sein kann. Die Signale dieses Sensors 152 können beispielsweise zur Steuerung 150 geführt sein. Beispielsweise kann auf diese Weise ein Kippwinkel $\alpha$ der Sperrklinke 148, beispielsweise eines Sperrklinkenhebels erfasst werden und zum Beispiel als Triggersignal für eine verzögerte Stoppfunktion des Antriebs 124 verwendet werden. Wird beispielsweise ein Ratschenzalan 146 erreicht, so kann ein Weiterdrehen des Antriebs 124 um einen vorgegebenen Winkel erfolgen, welcher der Bandfreigabe S entsprechen kann. Dieser Winkel, um welchen weitergespult wird, kann auch variabel gestaltet werden, beispielsweise um unterschiedliche Füllgrade des Schlechtwickels 122 und damit eine unterschiedliche Umsetzung von Drehwinkeln in abgespultes Trägerband 116 zu berücksichtigen. Alternativ oder zusätzlich zur Detektion eines Winkels der Sperrldinke 148 lassen sich auch zahlreiche andere Messgrößen fassen. Beispielsweise ließe sich mittels eines akustischen Sensors ein Klickgeräusch der Ratsche für eine derartige Steuerung nutzen. Wiederum alternativ oder zusätzlich ließen sich auch beispielsweise optische Sensoren einsetzen.

Die Steuerung der Bandfreigabevorrichtung 142 erfolgt in dieser Ausgestaltung in der Regel lediglich über den Drehwinkel des Schlechtwickels 122. Wie oben angedeutet, kann es daher zu unterschiedlichen Schlechtwickel-Bandreserven in Abhängigkeit vom Umfang des bereits aufgewickelten Bandmaterials des Trägerbands 116 kommen. Diesem Nachteil kann beispielsweise dadurch begegnet werden, dass der Schlupf auf den kleinsten Winkeldurchmesser angepasst wird, was dem Zustand eines noch unbenutzten Magazins 110 entspricht. Alternativ kann, wie oben beschrieben, der eingestellte Schlupf in diesem oder auch in anderen Ausführungsbeispielen auch an den Benutzungsgrad des Magazins 110 angepasst werden, um unterschiedliche Aufwicklungsgrade zu berücksichtigen.

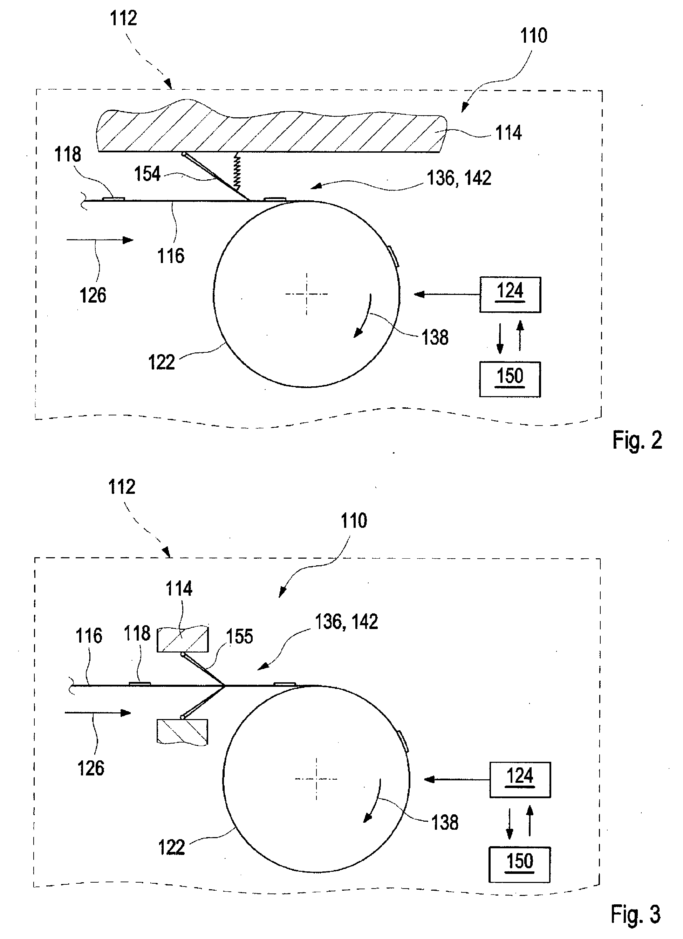

In Figur 2 ist ein zweites Prinzip einer Realisierung einer Bandfreigabevorrichtung 142 dargestellt. Dieses Ausführungsbeispiel zeigt wiederum eine Bandfreigabevorrichtung 142, welche zumindest teilweise in die Rückdrehsperre 136 integriert ist. Gleichzeitig zeigt dieses Ausführungsbeispiel, dass die Rückdrehsperre 136 nicht notwendigerweise am Schlechtwickel 122 ausgebildet sein muss. So zeigt Figur 2 anstelle einer drehwinkelabhängigen Sperre 144, dass auch die Tatsache ausgenutzt werden kann, dass die analytischen Hilfsmittel 118, beispielsweise die Lanzetten, aufgrund ihrer Dicke einen mechanischen Widerstand darstellen können. Dementsprechend ist in Figur 2 als Rückdrehsperre 136 eine federbelastete Klappe 154 auf der Eingangsseite des Schlechtwickels 122 vorgesehen. Diese gibt in Spulrichtung 126 nach und lässt das analytische Hilfsmittel 118 passieren. Die federbelastete Klappe 154 liegt jedoch im geschlossenen Zustand auf dem Trägerband 116 auf. In der rückwärtigen Richtung, also bei einer Bewegung des Trägerbands 116 entgegen der Spulrichtung 126, wirkt die Klappe somit als Anschlag, den das analytische Hilfsmittel 118, beispielsweise eine auf dem Trägerband 116 aufliegende Lanzette, nicht überwinden kann oder welche zumindest einen erheblichen Widerstand darstellt.

Die Steuerung 150 kann wiederum eingerichtet sein, um mit einer Funktion der federbelasteten. Klappe 154 gekoppelt zu werden. Beispielsweise kann wiederum, was in Figur 2 nicht dargestellt ist, ein Sensor 152 vorgesehen sein, welcher das Aufschwingen der federbelasteten Klappe 154 als Triggersignal für eine verzögerte Stoppfunktion für den Antrieb 124 verwendet. Nach einem Aufschwingen oder erneuten Schließen der federbelasteten Klappe 154 kann dann noch um einen vorgegebenen Betrag weitergespult werden, welcher wiederum im Wesentlichen der Bandreserve S entsprechen kann.

Die Funktion der federbelasteten Klappe 154 in Figur 2 kann auch durch andere federbelastete Elemente genutzt werden, was auch in sich selbst federnde Elemente einschließt. So zeigt Figur 3 ein Ausführungsbeispiel ähnlich zum Ausführungsbeispiel gemäß Figur 2, in welchem anstelle der federbelasteten Klappe 154 eine flexible Dichtlippe 155 auf der Eingangsseite des Schlechtwickels 122 verwendet wird. Diese Dichtlippe 155 gibt in Spulrichtung 126 nach und lässt die analytischen Hilfsmittel 118, beispielsweise die Lanzetten und/oder Testelemente, passieren. In der entgegengesetzten Richtung wird die Dichtlippe 155 jedoch mechanisch plastisch und/oder vorzugsweise elastisch verformt und erhöht damit innerhalb weniger Millimeter die Reibkraft des Trägerbands 116 derart, dass der rückdrehende Wickelantrieb 124 gestoppt wird. Die Stoppwirkung kann somit aufgrund zweierlei Mechanismen erfolgen, welche auch in Kombination einsetzbar sind. So kann zum einen ein rein mechanisches Anstoßen eines sich in Rückwärtsrichtung bewegenden analytischen Hilfsmittels 118 an der Dichtlippe 155 erfolgen, ähnlich zu einem Anstoßen an der federbelasteten Klappe 154 in Figur 2. Alternativ oder zusätzlich kann jedoch auch das Trägerelement 116 selbst blockieren, beispielsweise indem, wie oben beschrieben, die Dichtlippe 155 entgegen ihrer Öffnungsrichtung bewegt wird und sich verformt, wodurch eine Reibungskraft auf das Trägerband 116 erhöht wird.

Ein Vorteil der in den Figuren 2 und 3 sowie auch in der nachfolgenden Figur 4 beschriebenen Ausgestaltungen liegt darin, dass die Bandreserve S vom aktuellen Wickeldurchmesser, d.h. vom Aufspulgrad auf dem Schlechtwickel 122, unabhängig ausgestaltet werden kann. Für die Erhöhung der Reibkraft ist lediglich der tatsächlich zurückgelegte Bandweg in Figur 3 entscheidend.

Als weiteres alternatives Ausführungsbeispiel zu den Figuren 1 bis 3 zeigt Figur 4 ein als Kassette ausgestaltetes Magazin mit einer doppelten Gummiwalze 156. Das Trägerband 116 wird durch einen Walzenspalt 158 zwischen den Gummiwalzen 156 geführt. Die Gummiwalzen 156 sind derart eingerichtet, dass diese nur in Spulrichtung 156 drehen und in entgegengesetzter Richtung blockieren. Das nachgiebige Gummimaterial lässt die analytischen Hilfsmittel 118, beispielsweise die Lanzetten, in Spulrichtung 126 passieren. Beim Zurückdrehen des Schlechtwickels 122, also bei einem Transport des Trägerbands 116 entgegen der Spulrichtung 126, verdichtet sich jedoch das blockierende Gummi und sorgt schnell für eine ansteigende Reibkraft. Hierdurch wird der Wickelantrieb 124 nach wenigen Millimetern angehalten.

In Figur 5 ist ein Ausführungsbeispiel eines erfindungsgemäßen Magazins 110 und eines analytischen Testgeräts 112 gezeigt, welches auch mit den übrigen Ausführungsbeispielen kombiniert werden kann. Das Magazin 110 in diesem Ausführungsbeispiel kann beispielsweise analog zu den Magazinen 110 in den Ausführungsbeispielen gemäß den Figuren 1 bis 4 ausgestaltet sein. Auch eine Ausgestaltung gemäß dem Ausführungsbeispiel in Figur 6 oder gemäß anderen Ausführungsbeispielen ist grundsätzlich möglich. Dementsprechend weist das Magazin 110 mindestens eine Rückdrehsperre 136 auf, welche in Figur 5 nicht im Detail dargestellt ist und welche beispielsweise gemäß einer oder mehreren der oben und im Folgenden beschriebenen erfindungsgemäßen Ausführungsformen der Rückdrehsperre 136 ausgestaltet sein kann. Beispielsweise kann das Magazin 110 gemäß dem in Figur 1 dargestellten Magazin ausgestaltet sein und eine in den Schlechtwickel 122 integrierte Rückdrehsperre 136 umfassen. Alternativ oder zusätzlich sind jedoch auch andere Ausgestaltungen der Rückdrehsperre 136 und/oder sonstiger Bestandteile des Magazins 110 möglich.

In dem in Figur 5 dargestellten Ausführungsbeispiel umfasst das Magazin 110 zusätzlich eine oder mehrere Bremsen 160. Diese Bremsen 160 können beispielsweise auf das Trägerband 116 am Schlechtwickel 122, auf den Schlechtwickel 122 selbst oder auf den Gutwickel 120 oder das Trägerband 116 auf dem Gutwickel 120 einwirken. Auch eine Kombination der genannten Möglichkeiten ist denkbar. Das Ausführungsbeispiel umfasst also ein Magazin 110, bei welchem das Trägerband 116 zusätzlich durch eine integrierte Bremse 160 gesichert ist. Die Bremse 160 ist jedoch vorzugsweise ganz oder teilweise getrennt von der Rückdrehsperre 136, welche in Figur 5 nicht im Detail dargestellt ist, ausgestaltet. Die Bremse 160 kann ebenfalls eine gewisse Rückdrehhemmung ausüben, beispielsweise auf den Schlechtwickel 122. Diese Hemmung sollte jedoch mittels eines, vorzugsweise geringen, Kraftaufwandes leicht überwindbar sein. Die Bremse 160 kann somit beispielsweise eine dämpfende Eigenschaft auf das Magazin 110 und/oder das Trägerband 116 ausüben und/oder kann ein übermäßiges Abwickeln des Trägerbands 116 verhindern.

Die Bremse 160 übt eine Reibkraft auf das Trägerband 116 und/oder einen oder beide der Wickel 120, 122 aus. Diese Reibkraft dämpft ein übermäßiges Abwickeln von Bandmaterial. Falls die Bremse 160 auf beide Wickel 120, 122 wirkt, übt die reduzierte Reibung diese positive Dämpfung auch auf beide Wickel 120, 122 aus.

Die in den Figuren 1 bis 5 gezeigten Ausführungsbeispiele zeigen, dass eine in die Rückdrehsperre 136 integrierte Bandfreigabevorrichtung 142 beispielsweise in Form einer Ratsche, einer drehwinkelabhängigen Sperre, einer Reib-Dreh-Sperre oder in Form eines Elements realisiert werden kann, dessen Reibung auf das Trägerband 116 und/oder einen oder beide der Wickel 120, 122 von einer Drehrichtung und/oder einer Transportrichtung des Trägerbands 116 abhängig ist. In den Figuren 6A bis 6C ist hingegen ein Ausführungsbeispiel gezeigt, bei welchem die Bandfreigabevorrichtung 142 unabhängig von der Rückdrehsperre 136 realisiert werden kann. Wie oben bereits erläutert, handelt es sich bei diesem Ausführungsbeispiel um ein Magazin 110 mit einer beweglich gelagerten Bandumlenkung 140, welche im Folgenden auch als "Tänzer" bezeichnet wird. In Abwandlung des bereits oben beschriebenen Prinzips zur Realisierung einer geräteseitigen Bandfreigabe kann somit auch mindestens ein Tänzer in das Magazin 110, vorzugsweise in die Kassette integriert werden. Dieser Tänzer 140 ist vorzugsweise ausschließlich auf der Schlechtwiekel-Seite des Magazins 110 vorgesehen, also auf der dem Schlechtwickel 122 zuweisenden Seite des Trägerbands 116, von der Applikationsposition 128 aus betrachtet. Dieser Tänzer 140 verursacht einen variablen Umweg für den Bandverlauf des Trägerbands 116. Der Tänzer 140 ist dabei vorzugsweise mit einer Federkraft eines Federelements 162 beaufschlagt. Das Federelement 162 kann auch als Rückstellfeder wirken. Das Federelement 162 kann somit den Tänzer 140 stets mit einer Kraft beaufschlagen, um diesen in seine Ausgangslage zurückzudrücken. Diese Ausgangslage ist in Figur 6A dargestellt und zeigt eine Ruheposition mit maximaler Auslenkung des Tänzers 140.

Durch den Transportvorgang des Trägerbands 116, welcher in Figur 6B dargestellt ist, wird eine Zugkraft auf das Trägerband 116 und den Tänzer 140 ausgeübt, die den Tänzer 1140 zu einem teilweisen Einschwenken entgegen der Kraft des Federelements 162 veranlasst. Dies ist in Figur 6B dargestellt. Es handelt sich hierbei um eine Position des Tänzers 140, welche auch als Bandtransportposition bezeichnet werden kann und bei welcher der Tänzer 140 eine mittlere Auslenkung aufweist. Hierdurch bleibt die Zugspannung am Trägerband 116 konstant. Am Ende des Transportvorgangs stoppt der Wickelmotor, und ein frisches analytisches Hilfsmittel 118 befindet sich in der Applikationsposition 128. Beispielsweise kann hier eine frische Lanzette von einem nicht dargestellten Lanzettengreifer des Aktors 132 erfasst werden, beispielsweise indem eine frische Lanzette an einem Anschlag des Aktors 132 bzw. dessen Greifers anschlägt. Damit bleibt die Zugspannung erhalten, und der Tänzer 140 verbleibt ausgelenkt.

In Figur 6C ist schließlich eine Situation während der Hubbewegung 134 dargestellt. Beispielsweise kann es sich bei dieser Hubbewegung um eine Stichbewegung oder eine Probennahmebewegung handeln. Bei dieser Hubbewegung 134 wird durch die Auslenkung des Trägerbands 116 eine zusätzliche Zugkraft auf das Band und damit auch auf den Tänzer 140 ausgeübt. Der Tänzer 140 schwenkt weiter ein und nimmt dabei eine Hubposition mit einer minimalen Auslenkung ein, also einer Auslenkung, welche während des gesamten übrigen Prozesses nicht unterschritten wird. Hierbei gibt der Tänzer 140 die Bandreserve S frei. Die Federkonstante ist dabei vorzugsweise so auszulegen, dass bei normaler Zugspannung der Tänzer 140 zumindest näherungsweise etwa hälftig auslenkt, also beispielsweise mit einer Abweichung von nicht mehr als 20%, vorzugsweise nicht mehr als 10% und besonders bevorzugt von nicht mehr als 5% von einer hälftigen Auslenkung.

Ein großer Vorteil der Ausgestaltung gemäß den Figuren 6A bis 6C besteht darin, dass der Antrieb 124, beispielsweise der Wickelmotor, keine Rückdrehbewegung ausführen muss. Zusätzlich löst die Ausführung das Problem der Bandlose nach einer Hubbewegung 134, denn der maximal ausgelenkte Tänzer 140 fängt diese Bandlose durch die Rückstellkraft seines Federelements 162 wieder auf.

Auch gegenüber der oben bereits beschriebenen WO 2009/030359 A1 weist das analytische Testgerät 112 mit dem Magazin 110 gemäß den Figuren 6A bis 6C zahlreiche Vorteile auf. So kann hier insbesondere eine permanent wirkende Rückdrehsperre 136 realisiert werden, im Gegensatz zu bekannten Rückdrehsperren, welche bei Einsätzen des Magazins 110 in das analytische Testgerät 112 entsperrt werden.

Weiterhin erfolgt vorzugsweise bei dem Ausführungsbeispiel gemäß den Figuren 6A bis 6C eine asymmetrische Ausgestaltung der Bandfreigabevorrichtung 142 in Form des Tänzers 140. In WO 2009/030359 A1 werden beweglich gelagerte Bandumlenkungen sowohl auf der Gutwickelseite als auch auf der Schlechtwickelseite des Magazins vorgesehen, um eine symmetrische Auslenkung des Bandmaterials zu gewährleisten. Dies führt jedoch in der Praxis unter Umständen dazu, dass der Tänzer 140 nicht in der Lage ist, die zusätzliche Bandlose vom Gutwickel nach der Hubbewegung 134 zu kompensieren. Weiterhin lehrt WO 2009/030359 A1 als alternative Möglichkeit der Bandfreigabe einen aktiven Rückwärtsdrehschritt des Antriebs 124 während der Stechbewegung, wodurch jedoch nach der Stechbewegung das Problem einer kompletten Bandlose auftreten kann. Bei dem vorgeschlagenen Ausführungsbeispiel der vorliegenden Erfindung gemäß den Figuren 6A bis 6C ist hingegen die Bandfreigabevorrichtung 142 lediglich auf der Schlechtwickel-Seite vorgesehen, wohingegen auf der Gutwickel-Seite keine zusätzliche Bandfreigabevorrichtung 142, mit Ausnahme des Gutwickels 120 selbst, vorgesehen ist. Der Tänzer 140 löst die oben beschriebene Problematik auf elegante Weise. So ist dieser durch den Bandtransport bereits teilweise eingefedert, wie in Figur 6B zu erkennen ist. Bei der Hubbewegung 134, beispielsweise beim Stich, wird das Trägerband 116 allgemein vorzugsweise im Aktor 132 fixiert, beispielsweise in einem Halter des Greifers. Auf diese Weise sind beide Wickelseiten unabhängig voneinander zu betrachten. Auf der Gutwickel-Seite wirkt vorzugsweise der Freilauf des Gutwickels 120 und gibt entsprechend die Bandreserve G frei. Auf der Schlechtwickel-Seite hingegen federt der Tänzer 140 noch weiter ein und erzeugt hier die nötige Bandreserve S. Nach Beendigung des Stichs bzw. der Hubbewegung 134 wird das Trägerband 116 vom Aktor 132, beispielsweise vom Greifer und/oder Halter des Aktors 132, vorzugsweise entkoppelt, und der Tänzer 140 kann sich vollständig entspannen (siehe Figur 6A) und dabei die komplette Bandfreigabe wieder aufnehmen.

Insgesamt zeigen somit die Ausführungsbeispiele in den Figuren 1 bis 6C Wege auf, um trotz vorhandener Rückdrehsperre 136 im Magazin 110 eine ausreichende Bandreserve S auf der Schlechtwickel-Seite bereitstellen zu können. Insbesondere die in den Figuren 6A bis 6C dargestellte Ausführungsvariante ist eine besonders vorteilhafte Variante, da diese keinen rückdrehenden Wickelantrieb 124 benötigt und somit eine vergleichsweise wenig komplexe Ausgestaltung des analytischen Testgeräts 112 erfordert. Des Weiteren ist diese Ausführungsform in der Lage, die Bandfreigabe der Schlechtwickel-Seite nach der Hubbewegung 134 wieder einzuholen und damit die Bandführung zu optimieren. Bevorzugt ist auch die Ausgestaltung mit der Bremse 160 in Figur 5, welche auch mit den anderen Ausgestaltungen kombiniert werden kann, da diese ein übermäßiges Abwickeln des Trägerbands 116 verhindert, insbesondere während oder nach der Hubbewegung 134.

Bezugszeichenliste

| 110 | Magazin |
|-----|---------|
| 112 | analytisches Testgerät |
| 114 | Magazingehäuse |
| 116 | Trägerband |
| 118 | analytische Hilfsmittel |
| 120 | Gutwickel |
| 122 | Schlechtwickel |
| 124 | Antrieb |
| 126 | Spulrichtung |
| 128 | Applikationsposition |
| 130 | Durchbruch |
| 132 | Aktor |
| 134 | Hubbewegung |
| 136 | Rückdrehsperre |
| 138 | Aufwickelrichtung |
| 140 | beweglich gelagerte Bandumlenkung |
| 142 | Bandfreigabevorrichtung |
| 144 | drehwinkelabhängige Sperre |
| 146 | Ratschenzähne |
| 148 | Sperrklinke |
| 150 | Steuerung |
| 152 | Sensor |
| 154 | federbelastete Klappe |
| 155 | Dichtlippe |
| 156 | Gummiwalze |
| 158 | Walzenspalt |
| 160 | Bremse |
| 162 | Federelement |

**Patentansprüche**

1. Magazin (110) zum Einsatz in einem analytischen Testgerät (112), wobei das Magazin (110) als austauschbares Magazin (110) ausgestaltet ist, wobei das Magazin (110) eine Mehrzahl von analytischen Hilfsmitteln (118) auf einem Trägerband (116) umfasst, wobei die analytischen Hilfsmittel (118) mittels des Trägerbands (116) in mindestens einer Applikationsposition (128) des Magazins (110) bereitstellbar sind, wobei das Magazin (110) weiterhin mindestens einen Gutwickel (120) zur Aufnahme von Bereichen des Trägerbands (116) mit unverbrauchten analytischen Hilfsmitteln (118) aufweist, wobei das Magazin (110) weiterhin mindestens einen Schlechtwickel (122) zur Aufnahme von Bereichen des Trägerbands (116) mit verbrauchten analytischen Hilfsmitteln (118) aufweist, wobei das Trägerband (116) von dem Gutwickel (120) zu dem Schlechtwickel- (122) in einer Spulrichtung (126) bewegbar ist, wobei das Magazin (110) eine Rückdrehsperre (136) des Schlechtwickels (122) aufweist, wobei das Magazin (110) weiterhin eine Bandfreigabevorrichtung (142) aufweist, wobei die Bandfreigabevorrichtung (142) eingerichtet ist, um auf der dem Schlechtwickel (122) zuweisenden Seite der Applikationsposition (128) eine Schlechtwickel-Bandreserve des Trägerbands (116) bereitzustellen,
**dadurch gekennzeichnet, dass**
die Bandfreigabevorrichtung (142) lediglich auf der dem Schlechtwickel (122) zuweisenden Seite der Applikationsposition (128) vorgesehen ist, wobei das Magazin (110) derart ausgestaltet ist, dass ein analytisches Hilfsmittel (118), welches sich in der Applikationsposition (128) befindet, durch mindestens eine Fixiervorrichtung, insbesondere durch mindestens einen Greifer, fixierbar ist.

**2.** Magazin (110) nach einem der vorhergehenden Ansprüche, wobei eine Hubbewegung (134) eines in der Applikationsposition (128) befindlichen analytischen Hilfsmittels (118) einen maximalen Hub aufweist, insbesondere einen maximalen Hub von 2 mm bis 10 mm und vorzugsweise von 3 mm bis 6 mm, wobei die Schlechtwickel-Bandreserve 0,2 bis 0,8 des maximalen Hubs beträgt, vorzugsweise 0,5 des maximalen Hubs.

**3.** Magazin (110) nach einem der vorhergehenden Ansprüche, wobei das Magazin (110) weiterhin eingerichtet ist, um auf der dem Gutwickel (120) zuweisenden Seite der Applikationsposition (128) zusätzlich eine Gutwickel-Bandreserve des Trägerbands (116) bereitzustellen, wobei die Gutwickel-Bandreserve vorzugsweise im Wesentlichen der Schlechtwickel-Bandreserve entspricht.

**4.** Magazin (110) nach einem der vorhergehenden Ansprüche, wobei die Bandfreigabevorrichtung (142) weiterhin eingerichtet ist, um nach der Hubbewegung (134) des analytischen Hilfsmittels (118) die Schlechtwickel-Bandreserve zumindest teilweise wieder aufzunehmen.

**5.** Magazin (110) nach einem der vorhergehenden Ansprüche, wobei die Rückdrehsperre (136) eine permanent wirkende Rückdrehsperre (136) ist.

**6.** Magazin (110) nach einem der vorhergehenden Ansprüche, wobei die Rückdrehsperre (136) mindestens eine der folgenden Rückdrehsperren (136) umfasst: ein drehrichtungssensitives Element (144), insbesondere ein mit dem Schlechtwickel (122) verbundenes drehrichtungssensitives Element (144), wobei das drehrichtungssensitive Element (144) eine Drehung in einer Richtung ermöglicht und eine Drehung in einer anderen Richtung zumindest weitgehend verhindert; einen Freilauf, insbesondere einen mit dem Schlechtwickel (122) verbundenen Freilauf; eine Ratsche (144), insbesondere eine mit dem Schlechtwickel (122) verbundene Ratsche (144); eine Sperrklinke (148), insbesondere eine mit dem Schlechtwickel (122) verbundene Sperrklinke (148); eine auf das Trägerband (116) einwirkende drehrichtungsabhängige Bremse, insbesondere eine Walze (156) und/oder eine Dichtlippe (155); ein auf das Trägerband (116) einwirkendes federbelastetes Element (154); eine auf das Trägerband (116) einwirkende federbelastete Klappe (154); eine auf das Trägerband (116) einwirkende Dichtlippe (155).

**7.** Magazin (110) nach einem der vorhergehenden Ansprüche, wobei das Magazin (110) weiterhin mindestens eine Bremse (160) aufweist, wobei die Bremse (160) eingerichtet ist, um ein Rückspulen des Schlechtwickels (122) und/oder ein Vorwärtsspulen des Gutwickels (120) zu bremsen.

**8.** Magazin (110) nach einem der vorhergehenden Ansprüche, wobei die Bandfreigabevorrichtung (142) zumindest teilweise in die Rückdrehsperre (136) integriert ist.

**9.** Magazin (110) nach dem vorhergehenden Anspruch, wobei eine Funktion der Rückdrehsperre (136) von einer Position des Trägerbands (116) abhängig ist, wobei die Rückdrehsperre, (136) eingerichtet ist, um in einer Mehrzahl von Sperrpositionen eine Bewegung des Trägerbands (116) entgegen der Spulrichtung (126) zu verhindern, wobei zwischen den Sperrpositionen eine Bewegung des Trägerbands (116) entgegen der Spulrichtung (126) ermöglicht ist, bis eine nächste Sperrposition erreicht ist, wobei bei der Bewegung des Trägerbands (116) entgegen der Spulrichtung (126) die Schlechtwickel-Bandreserve zumindest teilweise freigegeben wird.

**10.** Magazin (110) nach einem der beiden vorhergehenden Ansprüche, wobei die Rückdrehsperre (136) ein drehrichtungssensitives Element (144), insbesondere ein mit dem Schlechtwickel (122) verbundenes drehrichtungssensitives Element (144), aufweist, wobei das drehrichtungssensitive Element (144) eine Drehung in einer Richtung ermöglicht, und eine Drehung in einer anderen Richtung zumindest weitgehend verhindert, wobei das drehrichtungssensitive Element (144) einen Totwinkel aufweist und eine Rückdrehung um den Totwinkel ermöglicht, wobei das Magazin (110) eingerichtet ist, um bei der Rückdrehung die Schlechtwickel-Bandreserve freizugeben.

**11.** Magazin (110) nach einem der drei vorhergehenden Ansprüche, wobei die Rückdrehsperre (136) mindestens ein auf das Trägerband (116) einwirkendes federbelastetes Element, insbesondere eine federbelastete Klappe (164), und/oder eine Dichtlippe (155) aufweist, wobei die analytischen Hilfsmittel (118) bei einer Bewegung des Trägerbands (116) in der Spulrichtung (126) das federbelastete Element und/oder die Dichtlippe (155) passieren können, wobei die analytischen Hilfsmittel (118) und/oder das Trägerband (116) bei einer Bewegung des Trägerbands (116) entgegen der Spulrichtung (126) an dem federbelasteten Element und/oder der Dichtlippe (155) blockieren.

**12.** Magazin (110) nach einem der vier vorhergehenden Ansprüche, wobei die Rückdrehsperre (136) mindestens eine Walze (156), insbesondere eine Doppelwalze, aufweist, wobei das Trägerband (116) durch einen durch die Walze (156) begrenzten Spalt geführt wird, wobei die Walze (156) eingerichtet ist, um bei einer Bewegung des Trägerbands (116) entgegen der Spulrichtung (126) eine Verformung zu durchlaufen, wobei durch die Verformung der Spalt verengt wird und eine weitere Bewegung des Trägerbands (116) zumindest erschwert wird.

13. Magazin (110) nach einem der vorhergehenden Ansprüche, wobei die Bandfreigabevorrichtung (142) zumindest teilweise unabhängig von der Rückdrehsperre (136) ausgebildet ist.

14. Magazin (110) nach dem vorhergehenden Anspruch, wobei die Bandfreigabevorrichtung (142) eine beweglich gelagerte Bandumlenkung (140) aufweist, wobei die beweglich gelagerte Bandumlenkung (140) zwischen der Applikationsposition (128) und dem Schlechtwickel- (122) vorgesehen ist.

15. Analytisches Testgerät (112), umfassend mindestens ein Magazin (110) nach einem der vorhergehenden Ansprüche, wobei das analytische Testgerät (112) eingerichtet ist, um einen Austausch des Magazins (110) zu ermöglichen, wobei das analytische Testgerät (112) einen Antrieb (124) zum Antreiben des Trägerbands (116) aufweist, wobei das analytische Testgerät (112) weiterhin eingerichtet ist, um mittels eines in der Applikationsposition (128) befindlichen analytischen Hilfsmittels (118) eine Hubbewegung (134) durchzuführen, insbesondere eine Stechbewegung und/oder eine Probennahmebewegung.

16. Analytisches Testgerät (112) nach dem vorhergehenden Anspruch, wobei das analytische Testgerät (112) weiterhin mindestens einen Sensor (152) zur Erkennung der Position des Trägerbands (116) aufweist, wobei das analytische Testgerät (112) weiterhin eine Steuerung (150) aufweist, wobei die Steuerung (150) eingerichtet ist, um die Schlechtwickel-Bandreserve entsprechend der erkannten Position des Trägerbands (116) einzustellen.

17. Analytisches Testgerät (112) nach dem vorhergehenden Anspruch, wobei das Magazin (110) derart eingerichtet ist, dass eine Funktion der Rückdrehsperre (136) von einer Position des Trägerbands (116) abhängig ist, wobei die Rückdrehsperre (136) eingerichtet ist, um in einer Mehrzahl von Sperrpositionen eine Bewegung des Trägerband (116) entgegen der Spulrichtung (126) zu verhindern, wobei zwischen den Sperrpositionen eine Bewegung des Trägerbands (116) entgegen der Spulrichtung (126) ermöglicht ist, bis eine nächste Sperrposition erreicht ist, wobei bei der Bewegung des Trägerbands (116) entgegen der Spulrichtung (126) die SchlechtwickelBandreserve freigegeben wird, wobei die Steuerung (150) eingerichtet ist, um bei Erreichen einer vorgegebenen Sperrposition einen Weitertransport des Trägerbands (116) um die Schlechtwickel-Bandreserve zu ermöglichen.

## Claims

1. Magazine (110) for use in an analytic test instrument (112), wherein the magazine (110) is embodied as replaceable magazine (110), wherein the magazine (110) comprises a plurality of analytic aids (118) on a carrier tape (116), wherein the analytic aids (118) can be made available in at least one application position (128) of the magazine (110) by means of the carrier tape (116), wherein the magazine (110) furthermore has at least one supply reel (120) for holding regions of the carrier tape (116) with unused analytic aids (118), wherein the magazine (110) furthermore has at least one take- up reel (122) for holding regions of the carrier tape (116) with used- up analytic aids (118), wherein the carrier tape (116) can be moved in a spooling direction (126) from the supply reel (120) to the take- up reel (122), wherein the magazine (110) has a rewind lock (136) of the take- up reel (122), wherein the magazine (110) furthermore has a tape release device (142), wherein the tape release device (142) is configured to make available a take- up reel tape reserve of the carrier tape (116) on the side of the application position (128) facing the take- up reel (122),
**characterized in that**
the tape release device (142) is only provided on the side of the application position (128) facing the take- up reel (122), wherein the magazine (110) is configured such that an analytic aid (118) situated in the application position (128) can be fixed by means of at least one fixing device, more particularly by means of at least one gripper.

2. Magazine (110) according to one of the preceding claims, wherein a lifting movement (134) of an analytic aid (118) situated in the application position (128) has a maximum lift, in particular a maximum lift of 2 mm to 10 mm and preferably of 3 mm to 6 mm, wherein the take-up reel tape reserve is 0.2 to 0.8 of the maximum lift, preferably 0.5 of the maximum lift.

3. Magazine (110) according to one of the preceding claims, wherein the magazine (110) is furthermore configured to make additionally available a supply reel tape reserve of the carrier tape (116) on the side of the application position (128) facing the supply reel (120), wherein the supply reel tape reserve preferably substantially corresponds to the take-up reel tape reserve.

4. Magazine (110) according to one of the preceding claims, wherein the tape release device (142) is furthermore configured to hold once again, at least in part, the take-up reel tape reserve after the lifting movement (134) of the analytic aid (118).

5. Magazine (110) according to one of the preceding claims, wherein the rewind lock (136) is a permanently acting rewind lock (136).

6. Magazine (110) according to one of the preceding claims, wherein the rewind lock (136) comprises at least one of the following rewind locks (136): a rotational-direction sensitive element (144), more particularly a rotational-direction sensitive element (144) connected to the take-up reel (122), wherein the rotational-direction sensitive element (144) enables a rotation in one direction and at least largely prevents a rotation in another direction; a freewheel, more particularly a freewheel connected to the take-up reel (122); a ratchet (144), more particularly a ratchet (144) connected to the take-up reel (122); a pawl (148), more particularly a pawl (148) connected to the take-up reel (122); a rotational-directiondependent brake acting on the carrier tape (116), more particularly a roller (156) and/or a sealing lip (155); a spring-loaded element (154) acting on the carrier tape (116); a spring-loaded flap (154) acting on the carrier tape (116); a sealing lip (155) acting on the carrier tape (116).

7. Magazine (110) according to one of the preceding claims, wherein the magazine (110) furthermore has at least one brake (160), wherein the brake (160) is configured to brake rewinding of the take-up reel (122) and/or winding-on of the supply reel (120).

8. Magazine (110) according to one of the preceding claims, wherein the tape release device (142) is at least partly integrated into the rewind lock (136).

9. Magazine (110) according to the preceding claim, wherein one function of the rewind lock (136) is dependent on a position of the carrier tape (116), wherein the rewind lock (136) is configured to prevent a movement of the carrier tape (116) counter to the spooling direction (126) in a plurality of locking positions, wherein a movement of the carrier tape (116) counter to the spooling direction (126) is made possible between the locking positions until a next locking position is reached, wherein the take-up reel tape reserve is at least partly released when the carrier tape (116) moves counter to the spooling direction (126).

10. Magazine (110) according to one of the two preceding claims, wherein the rewind lock (136) has a rotational-direction sensitive element (144), more particularly a rotational-direction sensitive element (144) connected to the take-up reel (122), wherein the rotational-direction sensitive element (144) enables a rotation in one direction and at least largely prevents a rotation in another direction, wherein the rotational-direction sensitive element (144) has a dead angle and enables rewinding through the dead angle, wherein the magazine (110) is configured to release the take-up reel tape reserve during rewinding.

11. Magazine (110) according to one of the three preceding claims, wherein the rewind lock (136) has at least one spring-loaded element acting on the carrier tape (116), more particularly a spring-loaded flap (154), and/or a sealing lip (155), wherein the analytic aids (118) can pass the spring-loaded element and/or the sealing lip (155) when the carrier tape (116) moves in the spooling direction (126), wherein the analytic aids (118) and/or the carrier tape (116) jam against the spring-loaded element and/or the sealing lip (155) when the carrier tape (116) moves counter to the spooling direction (126).

12. Magazine (110) according to one of the four preceding claims, wherein the rewind lock (136) has at least one roller (156), more particularly a double roller, wherein the carrier tape (116) is routed through a gap delimited by the roller (156), wherein the roller (156) is configured to undergo a deformation when the carrier tape (116) moves counter to the spooling direction (126), wherein the deformation leads to a narrowing of the gap and further movement of the carrier tape (116) is at least impeded.

13. Magazine (110) according to one of the preceding claims, wherein the tape release device (142) is designed to be at least partly independent of the rewind lock (136).

14. Magazine (110) according to the preceding claim, wherein the tape release device (142) has a moveably mounted tape deflection (140), wherein the moveably mounted tape deflection (140) is provided between the application position (128) and the take-up reel (122).

15. Analytic test instrument (112), comprising at least one magazine (110) according to one of the preceding claims, wherein the analytic test instrument (112) is configured to enable a replacement of the magazine (110), wherein the analytic test instrument (112) has a drive (124) for driving the carrier tape (116), wherein the analytic test instrument (112) is furthermore configured to carry out a lifting movement (134) by means of an analytic aid (118) situated in the application position (128), more particularly a piercing movement and/or a sample-taking movement.

16. Analytic test instrument (112) according to the preceding claim, wherein the analytic test instrument (112) furthermore has at least one sensor (152) for identifying the position of the carrier tape (116), wherein the analytic test instrument (112) furthermore has a control (150), wherein the control (150) is configured to set the take-up reel tape reserve in accordance with the identified position of the carrier tape (116).

17. Analytic test instrument (112) according to the preceding claim, wherein the magazine (110) is configured such that a function of the rewind lock (136) is dependent on a position of the carrier tape (116), wherein the rewind lock (136) is configured to prevent a movement of the carrier tape (116) counter to the spooling direction (126) in a plurality of locking positions, wherein a movement of the carrier tape (116) counter to the spooling direction (126) is made possible between the locking positions until a next locking position is reached, wherein the take-up reel tape reserve is released when the carrier tape (116) moves counter to the spooling direction (126), wherein the control (150) is configured to enable onward transport of the carrier tape (116) equal to the take-up reel tape reserve when a predetermined locking position is reached.

## Revendications

1. Cassette (110) destinée à être utilisée dans un testeur d'analyse (112), la cassette (110) étant conçue en tant que cassette (110) remplaçable, la cassette (110) comprenant une pluralité d'outils d'analyse (118) sur un ruban support (116), les outils d'analyse (118) pouvant être mis à disposition au moyen du ruban support (116) dans au moins une position d'application (128) de la cassette (110), la cassette (110) comportant en outre au moins une bobine neuve (120) destinée à recevoir des zones du ruban support (116) avec des outils d'analyse (118) neufs, la cassette (110) comportant en outre au moins une bobine usagée (122) destinée à recevoir des zones du ruban support (116) avec des outils d'analyse (118) usagés, le ruban support (116) étant déplaçable de la bobine neuve (120) vers la bobine usagée (122) dans une direction de débobinage (126), la cassette (110) comportant un blocage anti-retour (136) de la bobine usagée (122), la cassette (110) comportant en outre un dispositif de libération du ruban (142), le dispositif de libération du ruban (142) étant prévu pour mettre à disposition sur la face de la position d'application (128) dirigée vers la bobine usagée (122) une réserve de ruban support (116) sur la bobine usagée,
**caractérisée en ce que**
le dispositif de libération du ruban (142) est prévu uniquement sur la face de la position d'application (128) dirigée vers la bobine usagée (122), la cassette (110) étant conçue de telle sorte qu'un outil d'analyse (118) qui se trouve dans la position d'application (128) puisse être fixé à l'aide d'au moins un dispositif de fixation, notamment d'au moins une griffe.

2. Cassette (110) selon l'une quelconque des revendications précédentes, un déplacement en levée (134) d'un outil d'analyse (118) se trouvant dans la position d'application (128) présentant une course maximale, notamment une course maximale de 2 mm à 10 mm et de préférence de 3 mm à 6 mm, la réserve de ruban sur la bobine usagée s'élevant à de 0,2 à 0,8 de la course maximale, de préférence à 0,5 de la course maximale.

3. Cassette (110) selon l'une quelconque des revendications précédentes, la cassette (110) étant prévue en outre pour mettre à disposition en supplément sur la face de la position d'application (128) dirigée vers la bobine neuve (120) une réserve de ruban support (116) pour la bobine neuve, la réserve de ruban pour la bobine neuve correspondant de préférence sensiblement à la réserve de ruban pour la bobine usagée.

4. Cassette (110) selon l'une quelconque des revendications précédentes, le dispositif de libération du ruban (142) étant prévu en outre pour reprendre au moins en partie la réserve de ruban pour la bobine usagée, après le déplacement en levée (134) de l'outil d'analyse (118).

5. Cassette (110) selon l'une quelconque des revendications précédentes, le blocage antiretour (136) étant un blocage antiretour (136) à effet permanent.

6. Cassette (110) selon l'une quelconque des revendications précédentes, le blocage antiretour (136) comprenant au moins l'un des blocages antiretour (136) suivants : un élément (144) sensible au sens de rotation, notamment un élément sensible au sens de rotation (144) relié à la bobine usagée (122), l'élément sensible au sens de rotation (144) permettant une rotation dans un sens et empêchant au moins amplement une rotation dans un autre sens ; une roue libre, notamment une roue libre reliée à la bobine usagée (122) ; une crémaillère (144), notamment une crémaillère (144) reliée à la bobine usagée (122) ; un cliquet de blocage (148), notamment un cliquet de blocage (148) relié à la bobine usagée (122) ; un frein dépendant du sens de rotation agissant sur le ruban support (116), notamment un cylindre (156) et/ou une lèvre d'étanchéité (155) ; un élément (154) contraint par ressort agissant sur le ruban support (116) ; un clapet (154) contraint par ressort agissant sur le ruban support (116) ; une lèvre d'étanchéité (155) agissant sur le ruban support (116).

7. Cassette (110) selon l'une quelconque des revendications précédentes, la cassette (110) comportant en outre au moins un frein (160), le frein (160) étant prévu pour freiner un rembobinage de la bobine usagée (122) et/ou un débobinage de la bobine neuve (120).

8. Cassette (110) selon l'une quelconque des revendications précédentes, le dispositif de libération du ruban (142) étant intégré au moins en partie dans le blocage antiretour (136).

9. Cassette (110) selon la revendication précédente, une fonction du blocage antiretour (136) dépendant d'une position du ruban support (116), le blocage antiretour (136) étant prévu pour empêcher dans une pluralité de positions de blocage un déplacement du ruban support (116) à l'encontre du sens de débobinage (126), entre les positions de blocage, un déplacement du ruban support (116) à l'encontre du sens de débobinage (126) étant possible jusqu'à l'atteinte d'une position de blocage suivante, lors du déplacement du ruban support (116) à l'encontre du sens de débobinage (126), la réserve de ruban de la bobine usagée étant libérée au moins en partie.

10. Cassette (110) selon l'une quelconque des deux revendications précédentes, le blocage antiretour (136) comportant un élément (144) sensible au sens de rotation, notamment un élément (144) sensible au sens de rotation relié à la bobine usagée (122), l'élément (144) sensible au sens de rotation permettant une rotation dans un sens et empêchant au moins sensiblement une rotation dans un autre sens, l'élément (144) sensible au sens de rotation présentant un angle mort et permettant une rotation arrière autour de l'angle mort, la cassette (110) étant prévue pour libérer la réserve de ruban de la bobine usagée lors de la rotation arrière.

11. Cassette (110) selon l'une quelconque des trois revendications précédentes, le blocage antiretour (136) comportant au moins un élément contraint par ressort agissant sur le ruban support (116), notamment un clapet (154) contraint par ressort et/ou une lèvre d'étanchéité (155), lors d'un déplacement du ruban support (116) dans la direction de débobinage (126), les outils d'analyse (118) pouvant surmonter l'élément contraint par ressort et/ou la lèvre d'étanchéité (155), lors d'un déplacement du ruban support (116) à l'encontre de la direction de débobinage (126), les outils d'analyse (118) et/ou le ruban support (116) se bloquant contre l'élément contraint par ressort et/ou la lèvre d'étanchéité (155).

12. Cassette (110) selon l'une quelconque des quatre revendications précédentes, le blocage antiretour (136) comportant au moins un cylindre (156), notamment un double-cylindre, le ruban support (116) étant guidé à travers une encoche délimitée par le cylindre (156), le cylindre (156) étant conçu de manière à traverser une déformation lors d'un déplacement du ruban support (116) à l'encontre de la direction de débobinage (126), l'encoche se rétrécissant du fait de la déformation et un déplacement supplémentaire du ruban support (116) étant au moins rendu plus difficile.

13. Cassette (110) selon l'une quelconque des revendications précédentes, le dispositif de libération du ruban (142) étant conçu en étant au moins en partie indépendant du blocage antiretour (136).

14. Cassette (110) selon la revendication précédente, le dispositif de libération du ruban (142) comportant un renvoi de ruban (140) logé de manière mobile, le renvoi de ruban (140) logé de manière mobile étant prévu entre la position d'application (128) et la bobine usagée (122).

15. Testeur d'analyse (112), comprenant au moins une cassette (110) selon l'une quelconque des revendications précédentes, le testeur d'analyse (112) étant prévu pour permettre un remplacement de la cassette (110), le testeur d'analyse (112) comportant un entraînement (124) pour entraîner le ruban support (116), le testeur d'analyse (112) étant prévu par ailleurs pour procéder au moyen d'un outil d'analyse (118) se trouvant dans la position d'application (128) à un déplacement en levée (134), notamment à un déplacement en piquage et/ou à un déplacement de prélèvement d'échantillon.

16. Testeur d'analyse (112) selon la revendication précédente, le testeur d'analyse (112) comportant en outre au moins un capteur (152) pour détecter la position du ruban support (116), le testeur d'analyse (112) comportant par ailleurs un système de commande (150), le système de commande (150) étant prévu pour régler la réserve de ruban de la bobine usagée en fonction de la position détectée du ruban support (116).

17. Testeur d'analyse (112) selon la revendication précédente, la cassette (110) étant prévue pour qu'une fonction du blocage antiretour (136) dépende d'une position du ruban support (116), le blocage antiretour (136) étant prévu pour empêcher dans une pluralité de positions de blocage un déplacement du ruban support (116) à l'encontre de la direction de débobinage (126), un déplacement du ruban support (116) à l'encontre de la direction de débobinage (126) étant possible jusqu'à l'atteinte d'une position de blocage suivante, lors du déplacement du ruban support (116) à l'encontre de la direction de débobinage (126), la réserve de ruban de la bobine usagée étant libérée, le système de commande (150) étant prévu pour permettre, à l'atteinte d'une position de blocage prédéfinie, un transport ultérieur du ruban support (116) de la valeur de la réserve de ruban de la bobine usagée.

Fig. 1

Fig. 2

Fig. 3

Fig. 4

Fig. 5

Fig. 6 A

Fig. 6 B

Fig. 6 C

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2803345 C2
- DE 19819407 A1
- WO 2006059232 A1
- WO 2008022999 A1
- EP 1690496 B1
- WO 2003071940 A1
- WO 03088835 A2
- EP 2039293 A1
- WO 2009030359 A1
- US 20060240403 A1